# EUROPÄISCHE PATENTSCHRIFT

(11) **EP 0 996 738 B1**
(45) Veröffentlichungstag und Bekanntmachung des Hinweises auf die Patenterteilung: **01.02.2006**
(21) Anmeldenummer: 98946268.4
(22) Anmeldetag: 31.07.1998
(51) Int. Cl.: C12N 15/86, C12N 15/70, A61K 48/00, A61K 31/70

(54) **REKOMBINANTER VEKTOR, DER INFEKTIÖSE VIRALE GENOMSEQUENZEN MIT EINER GRÖSSE VON MEHR ALS 100 KB ENTHÄLT, VERFAHREN ZU SEINER HERSTELLUNG UND VERWENDUNG**
RECOMBINANT VECTOR CONTAINING INFECTIOUS, VIRAL GENOME SEQUENCES GREATER THAN 100 KB, METHOD FOR PRODUCING SAME AND USE FOR THE MUTAGENESIS OF THE VIRAL SEQUENCES
VECTEUR RECOMBINE CONTENANT DES SEQUENCES DE GENOME VIRALES INFECTIEUSES D'UNE TAILLE SUPERIEURE A 100 KB, SON PROCEDE DE PRODUCTION ET SON UTILISATION POUR LA MUTAGENESE DES SEQUENCES VIRALES

(30) Priorität: 01.08.1997 DE 19733364
(43) Veröffentlichungstag der Anmeldung: 03.05.2000
(73) Patentinhaber: Koszinowski, Ulrich H., 80805 München (DE)
(72) Erfinder: KOSZINOWSKI, Ulrich, H., D-80805 München (DE); MESSERLE, Martin, D-82152 Planegg (DE); BRUNE, Wolfram, Dr., D-80337 München (DE); Dr. Gabriele Hahn, 80799 München (DE)
(74) Vertreter: Grünecker, Kinkeldey, Stockmair & Schwanhäusser Anwaltssozietät
(86) Internationale Anmeldenummer: PCT/EP1998/004816
(87) Internationale Veröffentlichungsnummer: WO 1999/006582

(56) Entgegenhaltungen:
- WO-A-96/15779
- SPAETE R. R. ET AL.: "Insertion and deletion mutagenesis of the human cytomegalievirus genome." PROC. NATL. ACAD. SCI. USA, Bd. 84, 1987, Seiten 7213-7217, XP002086760
- MESSERLE M. ET AL.: "Reconstitution of a recombinant cytomegalo virus from two fragments cloned into bacterial artificial chromosomes; mouse cytomegalo virus genome cloning in Escherichia coli bacterial artificial chromosome for use as a vector in gene therapy." JOURNAL OF MOLECULAR MEDICINE, Bd. 74, Nr. 4, 1996, Seite B8 XP002086653
- KETNER G. ET AL.: "Efficient manipulation of the human adenovirus genome as an infectious yeast artificial chromosome clone." PROC. NATL. ACAD. SCI. USA, Bd. 91, 1994, Seiten 6186-6190, XP002086654
- SHIZUYA H. ET AL.: "CLONING AND STABLE MAINTENANCE OF 300-KILOBASE-PAIR FRAGMENTS OF HUMAN DNA IN ESCHERICHIA COLI USING AN F-FACTOR-BASED VECTOR" PROCEEDINGS OF THE NATIONAL ACADEMY OF SCIENCES OF USA, Bd. 89, 1. September 1992, Seiten 8794-8797, XP000573603
- GAGE P. J. ET AL.: "A CELL-FREE RECOMBINATION SYSTEM FOR SITE-SPECIFIC INTEGRATION OF MULTIGENIC SHUTTLE PLASMIDS INTO THE HERPES SIMPLEX VIRUS TYPE 1 GENOME" JOURNAL OF VIROLOGY, Bd. 66, Nr. 9, September 1992, Seiten 5509-5515, XP000674658
- MESSERLE M. ET AL.: "Cloning and mutagenesis of a herpesvirus genome as an infectious bacterial artificial chromosome." PROCEEDINGS OF THE NATIONAL ACADEMY OF SCIENCES OF THE UNITED STATES OF AMERICA, Bd. 94, Nr. 26, 23. Dezember 1997, Seiten 14759-14763, XP002086655

## Beschreibung

Die vorliegende Erfindung betrifft rekombinante Vektoren, die infektiöse virale Genomsequenzen sowie Sequenzen eines Klonierungsvehikels enthalten, und ein Verfahren zur Herstellung dieser Vektoren. Ferner betrifft die vorliegende Erfindung die Verwendung solcher rekombinanter Vektoren, insbesondere zur Mutagenese der darin enthaltenen infektiösen viralen Genomsequenzen, sowie ein Verfahren zur Mutagenese dieser Sequenzen.

Verschiedene Viren mit großer DNA sind wichtige Pathogene, entweder für den Menschen oder für Tiere oder Pflanzen, wobei sie zum Teil zu schweren oder sogar tödlichen Krankheiten führen. Beispielhaft sei hier das Cytomegalievirus (CMV) genannt, das zu den wichtigen menschlichen pathogenen Viren zählt. CMV hat eine hohe Prävalenz in der Bevölkerung und kann zu schweren Krankheiten führen, insbesondere bei immunologisch unreifen Patienten oder bei Patienten mit einem beeinträchtigten Immunsystem (1). Da das menschliche Cytomegalievirus und das Maus-Cytomegalievirus (MCMV) eine Reihe von Ähnlichkeiten in ihrer Biologie und Pathogenese zeigen (2), wurde die Infektion von Mäusen mit MCMV zu einem intensiv untersuchten in vivo-Modell zur Untersuchung der Pathogenese der CMV-Infektion. Die ca. 235 kb große Genome von sowohl menschlichem als auch Mäuse-CMV sind die größten Genome von DNA-Viren der Säuger. Die Sequenzanalyse der menschlichen und Mäuse-CMV-Genome zeigt eine ähnliche genetische Organisation und eine Kodierungsfähigkeit für vermutlich mehr als 220 Polypeptide (3, 4, 5).

Die Größe des Genoms solcher Viren macht es schwierig, das gesamte Genom bzw. die für Replikation und Verpackung notwendigen Teile des Genoms zu klonieren, zu analysieren und gezielte Veränderungen vorzunehmen. Dies liegt unter anderem daran, daß die bis jetzt verwendeten Verfahren auf die Verwendung mehrfacher Rekombinationsschritte in eukaryontischen Zellen angewiesen sind, die 1. selten, 2. fehleranfällig und 3. praktisch nicht zu kontrollieren sind. Dadurch werden aufwendige Selektionsverfahren notwendig, um gewünschte Mutanten zu isolieren und charakterisieren zu können.
Verfahren zur Klonierung und Vermehrung von Fragmenten humaner genomischer DNA sind ebenfalls bekannt (Shizuya et al., PNAS USA 1992, 89: 8794-8797).
Informationen über die Funktion eines Großteils der CMV-Genprodukte liegen aufgrund der Größe der viralen DNA und der damit verbundenen schwierigen Manipulierbarkeit des viralen Genoms nach wie vor kaum vor, und CMV-Klone und CMV-Mutanten sind ihrer großen DNA und ihrer langsamen Replikationskinetik wegen bisher nur sehr schwierig zu erhalten. Dies steht z.B. im Gegensatz zu der Situation beim Herpes simplex Virus (α-Herpesviren) (6), das eingehend untersucht wurde und für das viele virale Genfunktionen geklärt werden konnten.

Für die Unterbrechung und Deletion von CMV-Genen wurde ein Verfahren zur Insertionsmutagenese in eukaryontischen Zellen entwickelt (7, 8). Aufgrund der geringen Häufigkeit homologer Rekombinationen in eukaryontischen Zellen ist das Verfahren jedoch sehr ineffektiv. Zusätzlich wurden häufig zufällige Deletionen und die Bildung nicht erwünschter rekombinanter Viren beobachtet (7, 9). Obwohl Selektionsverfahren dieses ursprüngliche Verfahren verbessert haben (9, 10, 11), bleibt die Erzeugung von CMV-Mutanten eine aufwendige und häufig erfolglose Aufgabe. Kürzlich konnte ein Verfahren zur Konstruktion von rekombinanten Herpesviren aus klonierten, überlappenden Fragmenten (12) auch auf CMV ausgedehnt werden (13; Messerie et al., J Mol Med 1996, 74(4): B8)
Dies ist bereits eine deutliche Verbesserung gegenüber den oben beschriebenen Verfahren, da dieses Verfahren nur rekombinante Viren erzeugt und eine Selektion gegen nicht rekombinante Wildtypviren überflüssig macht. Die resultierenden Mutanten sind jedoch immer noch das Produkt mehrerer Rekombinationsereignisse in eukaryontischen Zellen, die schwierig zu kontrollieren sind. Ein korrekter Wiederaufbau des viralen Genoms kann nur nach Wachstum und Isolierung der Virusmutante sichergestellt werden.

Aufgabe der vorliegenden Erfindung ist es, rekombinante Vektoren bereitzustellen, die es erlauben, das gesamte Genom oder zumindest die für die Replikation und Verpackung notwendigen Teile des Genoms eines Virus, dessen Genom größer als 100 kb, vorzugsweise größer als 200 kb, ist, in einen Organismus oder eine Zelle einzubringen, dort zu halten und zu vermehren sowie gezielte Veränderungen an den viralen Sequenzen vorzunehmen.

Diese Aufgabe wird durch einen rekombinanten Vektor gelöst, der infektiöse virale Genomsequenzen mit einer Größe von mehr als 100 kb, vorzugsweise von mehr als 200 kb, sowie Sequenzen eines Klonierungsvehikels enthält , die die Fähigkeit zur DNA-Replikation in einer Wirtszelle besitzen, wobei das Klonierungsvehikel ein bakterielles künstliches Chromosom (BAC) ist.
Der Ausdruck "infektiöse virale Genomsequenzen" umfaßt im Sinne der Erfindung sowohl das gesamte Genom als auch Teile des Genoms eines Virus, die für die Replikation und die Verpackung in einem Wirtsorganismus bzw. einer Wirtszelle unerläßlich sind.

Die infektiösen viralen Genomsequenzen des rekombinanten Vektors gemäß der Erfindung können von einem DNA-Virus stammen; vorzugsweise stammen sie von einem Herpesvirus, wobei besonders das menschliche Cytomegalievirus (ein wichtiges Pathogen für den Menschen) und das Maus-Cytomegalievirus genannt werden sollen. Weiterhin eignen sich Genomsequenzen von anderen DNA-Viren sowie alle Herpesvirusgenome, wie z.B. Herpes simplex Virus Typ 1 (Größe des Genoms: 152 kb) und 2 (155 kb), Epstein-Barr-Virus (172 kb), Varizella Zoster (125 kb), humane Herpesviren 6, 7 oder 8 (HHV6, 159 kb; HHV7, 145 kb; HHV8, ca. 160 kb), Herpesviren der Tiere, beispielsweise Pseudorabies-Virus (ca. 130 kb), Bovine Herpesviren 1 (135 kb), 2 (140 kb), 3 oder 4 (156 kb), und das murine Gammaherpesvirus 68 (MHV 68, ca. 140 kb).

Die im erfindungsgemäßen rekombinanten Vektor enthaltenen Sequenzen eines Klonierungsvehikels besitzen die Fähigkeit zur DNA-Replikation in geeigneten Wirtszellen oder -organismen und dienen als Träger der viralen Sequenzen, die passiv mitrepliziert und zusammen mit den Sequenzen des Klonierungsvehikels isoliert und gereinigt werden können. Es hat sich herausgestellt, daß eine niedrige Kopienzahl des erfindungsgemäßen rekombinanten Vektors in der Wirtszelle für die Stabilität der darin enthaltenen viralen Genomsequenzen vorteilhaft ist. Daher werden als Sequenzen eines Klonierungsvehikels solche Sequenzen bevorzugt eingesetzt, die von low-copy-Vektoren stammen.

Da der erfindungsgemäße rekombinante Vektor gemäß der Erfindung infektiöse Genomsequenzen enthält, kann er nach dem Einbringen in eine geeignete permissive Zelle wie ein Virusgenom repliziert und verpackt werden. Es hat sich jedoch herausgestellt, daß durch die auf die Anwesenheit von Sequenzen eines Klonierungsvehikels zurückzuführende Überlänge des Vektors (gegenüber der ursprünglichen Länge der viralen Genomsequenzen) die Verpackung der Viruspartikel beeinträchtigt wird. Daher ist es vorteilhaft, die Sequenzen des Klonierungsvehikels vor der Replikation und Verpackung der viralen Genomsequenzen aus dem rekombinanten Vektor zu entfernen. Zu diesem Zweck sieht die Erfindung in einer Ausführungsform vor, daß die Sequenzen des Klonierungsvehikels von identischen Sequenzabschnitten flankiert sind, die das Herausschneiden der Sequenzen durch homologe Rekombination ermöglichen.

In einer weiteren, besonders bevorzugten Ausführungsform des erfindungsgemäßen Vektors sind die Sequenzen des Klonierungsvehikels von Erkennungssequenzen für sequenzspezifische Rekombinasen und/oder von Restriktionsschnittstellen flankiert, die im restlichen Vektor nicht vorkommen. Zum Erhalt von infektiösen viralen Genomsequenzen, die frei von Sequenzen des Klonierungsvehikels sind, werden diese durch eine Rekombinase, wie z.B. die Rekombinase FLP, und/oder durch ein Restriktionsenzym herausgeschnitten. Besonders bevorzugt sind rekombinante Vektoren, bei denen die Erkennungssequenzen für sequenzspezifische Rekombinasen loxP-Stellen sind, die durch die Rekombinase Cre erkannt und geschnitten werden. Gegebenenfalls können die zu klonierenden viralen Genomsequenzen zunächst in eine geeignete Zelle eingebracht werden, wenn die sie enthaltende Zelle für die Klonierung ungeeignet sein sollte.

Vorzugsweise enthält der erfindungsgemäße Vektor Selektions- und/oder Markergene, beispielsweise gpt, das Hygromycin-Resistenz-Gen, das Neomycin-Resistenz-Gen, das green-fluorescent-Protein oder das lacZ-Gen, um die Selektion (Anreicherung) bzw. Identifikation (z.B. durch Färbetechniken) von rekombinante Vektoren enthaltenden Zellen zu erleichtern.

Die vorliegende Erfindung betrifft ebenfalls Zellen, die einen rekombinanten Vektor der oben beschriebenen Art enthalten.

Ferner ist Aufgabe der Erfindung, ein Verfahren zur Herstellung eines erfindungsgemäßen rekombinanten Vektors bereitzustellen, das die Klonierung großer, möglichst vollständiger, auf jeden Fall aber infektiöser (d.h. replikationsfähiger) viraler Genomsequenzen in eukaryontischen Zellen mit möglichst wenigen Rekombinationsschritten erlaubt, und bei dem eine wiederholte Isolierung und Selektion der Rekombinanten nicht erforderlich ist.

Diese Aufgabe wird gemäß der vorliegenden Erfindung durch ein Verfahren gelöst, das folgende Schritte umfaßt:
a) Einbringen einer Sequenz (1), enthaltend Sequenzen eines Klonierungsvehikels, in eine Zelle, die infektiöse virale Genomsequenzen enthält, und
b) Rekombinieren der Sequenz (1) aus Schritt a) mit den viralen Genomsequenzen, vorzugsweise über homologe Rekombination, zum Erhalt eines rekombinanten Vektors.

Das Verfahren ist insbesondere zur Klonierung solcher Genomsequenzen geeignet, die größer als 100 kb (vorzugsweise größer als 200 kb) sind, es kann jedoch auch zur Klonierung kleinerer DNA-Fragmente eingesetzt werden. Beispiele für virale Genomsequenzen, die nach dem Verfahren gemäß der Erfindung mit der Sequenz (1) rekombiniert werden können, schließen die Genome der Herpes simplex Virus Typ 1 (Größe des Genoms: 152 kb) und 2 (155 kb), des Epstein-Barr-Virus (172 kb), des Varizella Zoster-Virus (125 kb), der humanen Herpesviren 6, 7 oder 8 (HHV6, 159 kb; HHV7, 145 kb; HHV8, ca. 160 kb), der Herpesviren der Tiere, beispielsweise Pseudorabies-Virus (ca. 130 kb), Bovine Herpesviren 1 (135 kb), 2 (140 kb), 3 oder 4 (156 kb), und des murinen Gammaherpesvirus 68 (MHV 68, ca. 140 kb) ein. Die homologe Rekombination zwischen den viralen Sequenzen und der Sequenz (1) kann beispielsweise dadurch herbeigeführt werden, daß die Sequenz (1) Abschnitte enthält, die homolog zu den zu klonierenden viralen Genomsequenzen sind.

Vorzugsweise werden als Wirtszellen für den Schritt a) eukaryontische Zellen verwendet, beispielsweise Säugerzellen, Insektenzellen oder Hefezellen, wobei insbesondere primäre Fibroblasten vom Menschen (z.B. humane Vorhautfibroblasten (HFF)), NIH3T3-Fibroblasten (ATCC CRL1658) oder Maus-Fibroblasten verwendet werden können. Zum Einbringen der Sequenz (1) werden Wirtszellen ausgewählt, die für das jeweilige Virus permissiv sind, d.h. Zellen, die die Fähigkeit besitzen, das Wachstum dieses Virus zu unterstützen. Die Einbringung der Sequenz (1) in die eukaryontische Zelle erfolgt durch ein Calciumphosphat-Präzipitations-, Elektroporations- oder Lipofektionsverfahren oder mittels anderer Verfahren nach dem neuesten Stand der Technik. Die Sequenz (1) kann ebenfalls durch einen viralen Vektor in die Zelle eingebracht werden.

In einer besonders bevorzugten Ausführungsform des erfindungsgemäßen Herstellungsverfahrens wird als Zelle in Schritt a) ein bakterieller Organismus, vorzugsweise *E*. *coli,* verwendet. Das Einbringen der Sequenz (1) kann beispielsweise durch Elektroporation oder jedes andere im Stand der Technik bekannte Verfahren geschehen.

Ferner betrifft die Erfindung die Verwendung eines rekombinanten Vektors der oben beschriebenen Art zur Mutagenese der darin enthaltenen infektiösen viralen Genomsequenzen.

Eine weitere Aufgabe der vorliegenden Erfindung ist es, ein Verfahren bereitzustellen, mit dem Veränderungen an den im erfindungsgemäßen Vektor enthaltenen viralen Genomsequenzen vorgenommen werden können.

Diese Aufgabe wird durch ein Mutageneseverfahren gelöst, das folgende Schritte umfaßt:
A) Einbringen des rekombinanten Vektors in eine bakterielle Wirtszelle oder in Hefe; und
B) Mutagenisieren der viralen Genomsequenzen.

Die Mutagenese kann gezielt durch homologe Rekombination mit in der bakteriellen Wirtszelle oder Hefe enthaltenen DNA-Molekülen (z.B. als Shuttle-Plasmid) erfolgen, die die Mutation tragen, oder ungezielt ("random"), z.B. durch Transposon-Mutagenese. Unter "Mutagenese" im Sinne der Erfindung versteht man die Einführung jeder möglichen Veränderung des genetischen Materials, beispielsweise Deletionen, Insertionen, Substitutionen oder Punktmutationen.

Das erfindungsgemäße Mutageneseverfahren ist deswegen vorteilhaft, weil die Veränderungen in der bakteriellen Wirtszelle oder in Hefe ohne Selektionsdruck erfolgen: Es können also auch Mutationen in das Genom eingefügt werden, die einen Wachstumsnachteil (oder sogar einen letalen Phänotyp) für die Mutante zur Folge haben können. Bisherige Mutagenese-Verfahren für Herpesviren erlauben dies entweder gar nicht oder nur mit beträchtlichem Aufwand. Auf die späteren Eigenschaften der Virus-Mutante muß zunächst bei der Mutagenese keine Rücksicht genommen werden, da Herstellung des mutanten Genoms und Herstellung bzw. Charakterisierung der Virus-Mutante getrennte Prozesse sind.

Eine Transposon-Mutagenese klonierter Herpesvirus-Genome ist nützlich zur einfachen und effizienten Herstellung einer großen Anzahl von Mutanten, die u.a. für Screening-Verfahren dienen können. Durch eine saturierte Mutagenese läßt sich eine umfassende Sammlung ("Library") mit Mutationen in allen Genen und in allen regulatorischen Sequenzen generieren.

In einer vorteilhaften Durchführungsform des erfindungsgemäßen Mutageneseverfahren ist der rekombinante Vektor nach einem der oben beschriebenen Herstellungsverfahren erhältlich.

Die Erfindung betrifft ferner einen rekombinanten Vektor, der infektiöse, durch ein Mutageneseverfahren wie oben beschrieben veränderte virale Genomsequenzen mit einer Größe von mehr als 100 kb, vorzugsweise mehr als 200 kb, sowie Sequenzen eines Klonierungsvektors enthält.

Das Einbringen des mutagenisierten Vektors in permissive Zellen führt ausschließlich zur Bildung von einheitlichen Virus-Mutanten, da die eingebrachten Vektoren klonal sind d.h. sich auf eine bakterielle Zelle (Klon) bzw. auf ein Vektor-Molekül zurückführen lassen. Nachträgliche, in der Regel mühsame und langwierige Selektions- oder Anreicherungsverfahren für Mutanten - ein typischer Bestandteil bisheriger Mutagenese-Verfahren für große Virusgenome - sind deshalb nicht notwendig.

Sowohl mutagenisierte als auch nicht mutagenisierte rekombinante Vektoren gemäß der Erfindung eignen sich als Medikament, vorzugsweise zur Durchführung von somatischer Gentherapie, oder als Impfstoff.

Als Anwendungsmöglichkeiten für die erfindungsgemäßen Vektoren und Verfahren kommen unter anderem in Betracht:
- Charakterisierung von viralen Genen bzw. Funktionen und Proteinen z.B. mit dem Ziel essentielle Virus-Strukturen zu identifizieren, um Hemmstoffe (Medikamente) gegen Viren entwickeln zu können.
- Herstellung einer CMV-Vaccine bzw. auf CMV-basierende Vaccine gegen andere Krankheitserreger (Attenuierung des Virus durch Deletion von Genen, und Insertion von Genen, die für Antigene kodieren).
- Herstellung von Vektoren für die Gentherapie (Deletion von viralen Genen, Austausch gegen therapeutisch nützliche Trans-Gene).

Im folgenden werden die angefügten Zeichnungen beschrieben:

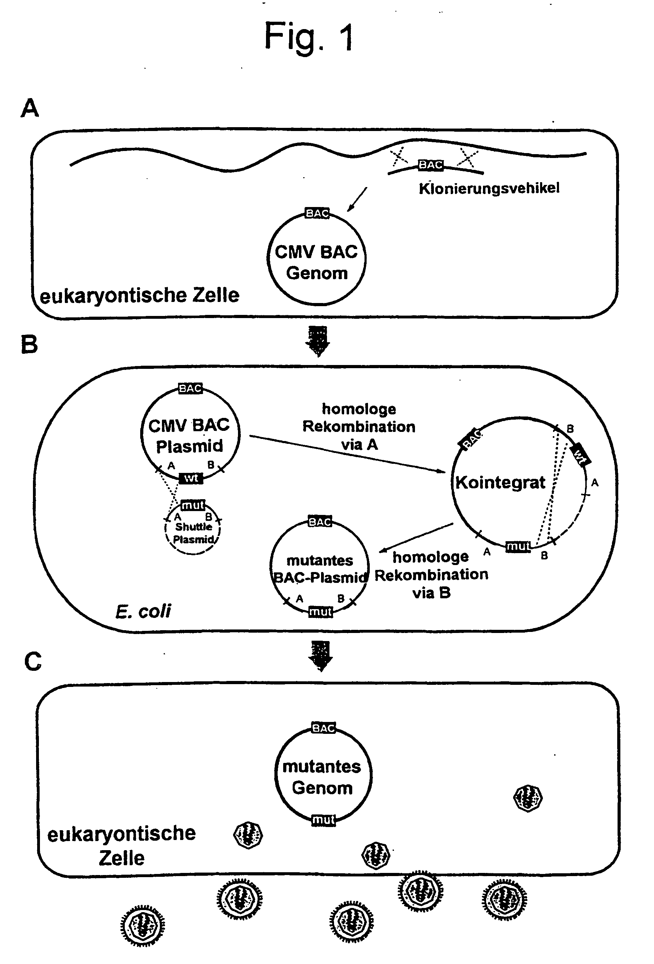

Figur 1 zeigt eine Strategie zur Klonierung und Mutagenese eines großen Virusgenoms unter Anwendung der erfindungsgemäßen Herstellungs- und Mutageneseverfahren.
   (A) Ein großes virales Genom und ein Rekombinationsplasmid mit bakteriellen Vektorsequenzen wurden in eukaryontische Zellen eingebracht, um eine rekombinante DNA (BAC) zu erzeugen. Dies kann durch verschiedene Verfahren erfolgen, z.B. Infektion, Transfektion, Elektroporation usw. Zirkuläre DNA wurde aus den infizierten Zellen isoliert und in *E. coli* übertragen. (B) Eine Mutagenese des BAC wurde in *E. coli* durch homologe Rekombination mit einem mutanten Allel durchgeführt, und das mutierte BAC wurde in eukaryontische Zellen transfiziert, um rekombinante Virus-Genome bzw. Viren zu rekonstituieren (C).

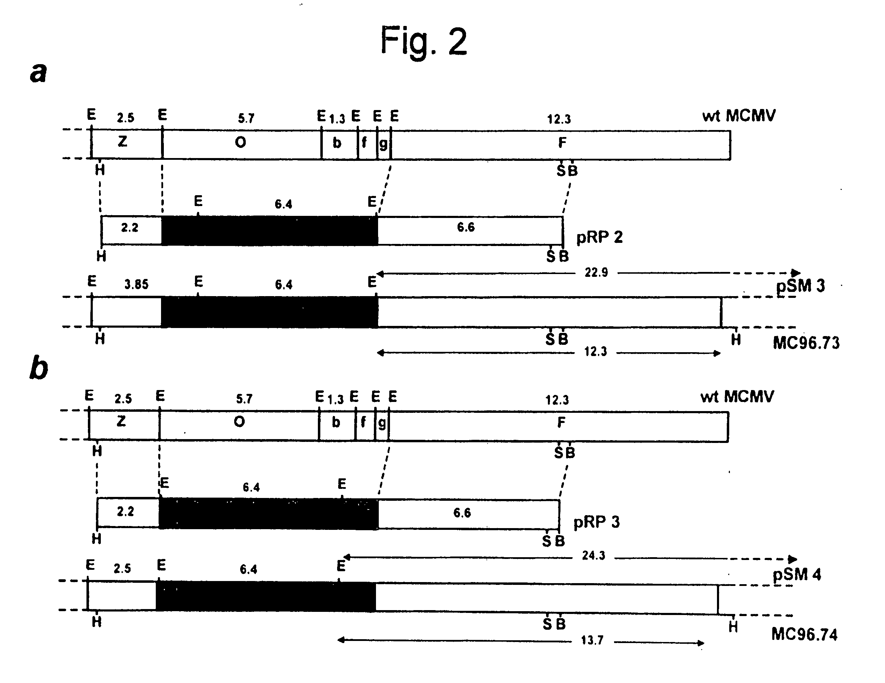

Figur 2 zeigt die Herstellung und den Aufbau der MCMV künstlichen bakteriellen Chromosome (BACs) pSM3 (a) und pSM4 (b) und die Genomstruktur der abgeleiteten rekombinanten Viren MC96.73 und MC96.74. Die EcoRI-Restriktionskarte des rechten terminalen Endes des Genoms des MCMV-Stamms Smith ist oben dargestellt. Die Rekombinationsplasmide pRP2 und pRP3 enthalten 2.2 und 6.6 kb flankierender MCMV homologer Sequenzen (weiße Kästen), den BAC-Vektor (grau) und das gpt-Gen (schraffiert), flankiert von loxP-Stellen (schwarz). Die Eco-RI-Restriktionskarte der BACs pSM3 und pSM4 ist darunter dargestellt. Die terminalen EcoRI-Fragmente des MCMV-Genoms sind in den BAC-Plasmiden pSM3 und pSM4 fusioniert, was zu neuen Fragmenten von 22,9 bzw. 24,3 kb führt. Die linearen Genome der rekombinanten Viren MC96.73 und MC96.74 enthalten terminale EcoRI-Fragmente mit einer Länge von 12,3 bzw. 13,7 kb. Zusätzliche Restriktionsenzymstellen sind mit BamHI (B), HindIII (H) und SfiI (S) angegeben.

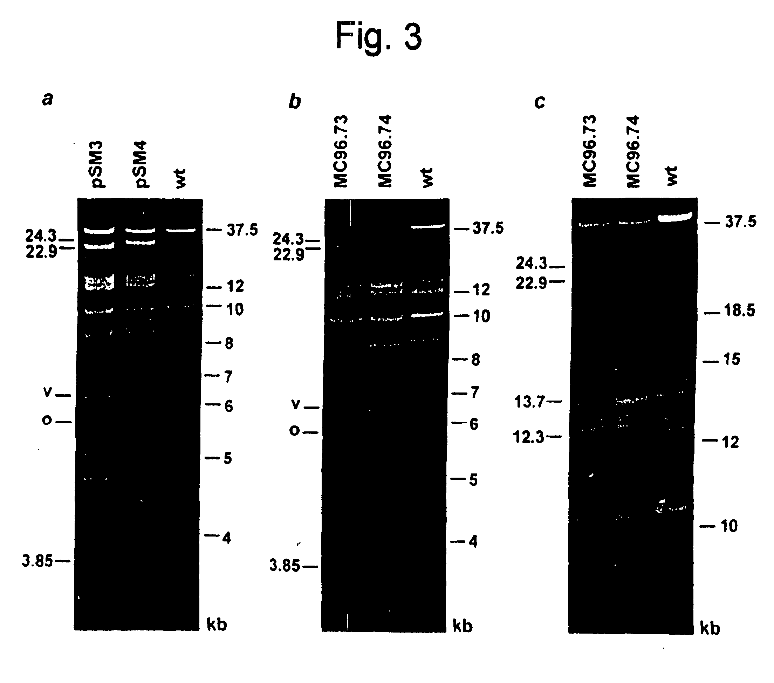

Figur 3 zeigt die Strukturanalyse der künstlichen bakteriellen Chromosome (BACs) pSM3 und pSM4 (a) und der rekonstituierten Virusgenome (b, c). (a) Ethidiumbromid-gefärbtes Agarosegel von EcoRI-verdauten BACs, die aus *E. coli*-Kulturen isoliert wurden (Spuren pSM3 und pSM4) und von Wildtyp-MCMV-DNA, isoliert aus gereinigten Virionen (Spur wt). (b) Restriktionsenzymanalyse der viralen MC96.73- und MC96.74-Genome. Die BACs pSM3 und pSM4 wurden in Maus-Embryonenfibroblasten transfiziert und der Überstand der transfizierten Zellen wurde verwendet, um neue Zellen zu infizieren. Die DNA, die aus den infizierten Zellen isoliert wurde und Wildtyp-MCMV-DNA wurde mit EcoRI verdaut und durch Elektrophorese auf 0,6 % Agarosegelen 14 Stunden aufgetrennt. Die EcoRI-O(O) und die Vektorfragmente (v) sind angegeben und die Größe der zusätzlichen Banden ist links gezeigt. (c) Auftrennung der EcoRI-Fragmente, die in (b) dargestellt sind, nach Elektrophorese für 28 Stunden.

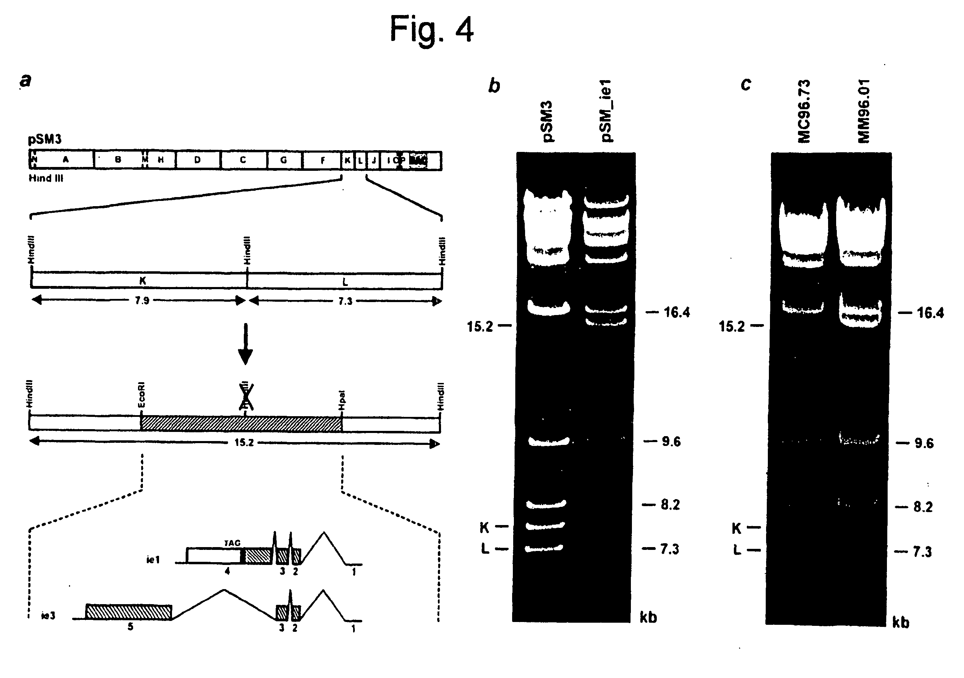

Figur 4 zeigt die Konstruktion der MCMV-ie1-Mutante MM96.01 (a), die Strukturanalyse des mutierten künstlichen bakteriellen Chromosoms (BACs) (b) und des Genoms der ie1-Mutanten MM96.01 (c). (a) Die Hindlll-Schnittstelle zwischen den HindIII-K- und -L-Fragmenten des Genoms des MCMV-Stamms Smith (oben) wurde unter Verwendung des EcoRI-HpaI-Fragments (schraffierter Bereich) durch Mutagenese entfernt. Die Exon-Intron-Struktur der ie1-und ie3-Gene ist angezeigt und die proteinkodierenden Sequenzen sind als schraffierte Kästchen dargestellt. Die Mutation führt zu einer Rasterverschiebung nach 273 Codons und der Bildung eines neuen Stoppcodons nach zusätzlichen 9 Codons (schwarzer Kasten). Der offene Kasten bezeichnet den Teil des offenen ie1-Leserahmens, der im mutierten Virus nicht translatiert wird. (b) Ethidiumbromid-gefärbtes Agarosegel des mit HindIII verdauten Eltern-BACs pSM3 und des mutierten BACs pSMie1. (c) HindIII-Muster der Genome des rekombinanten Virus MC96.73 und des ie1-Mutanten MM96.01. Das Hindlll-K- und -L-Fragment und das neue 15,2 kb-Fragment sind links angegeben und die Größe einiger HindIII-Fragmente ist am rechten Bildrand angegeben.

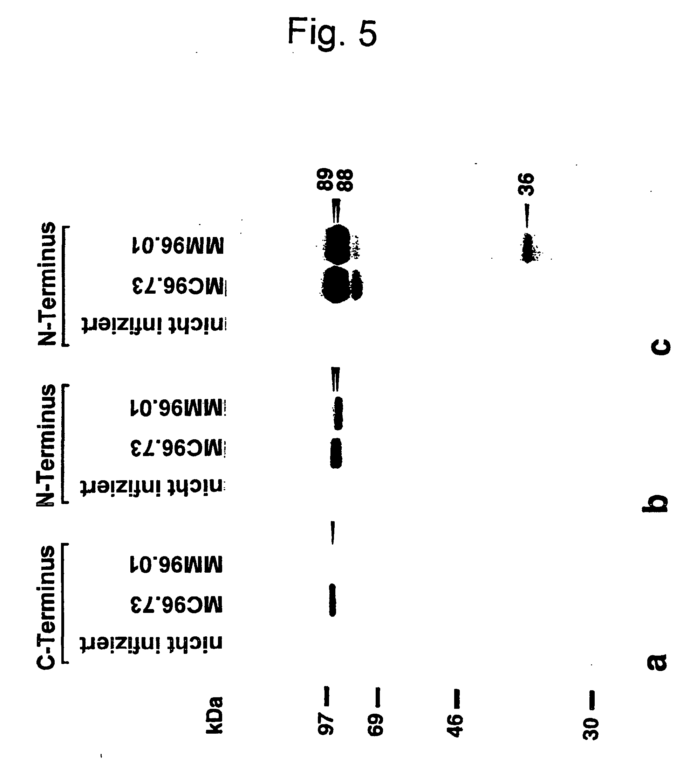

Figur 5 zeigt die Abwesenheit von pp89 in Zellen, die mit dem ie1-Mutanten MM96.01 infiziert sind. Embryonale Maus-Fibroblasten (MEF) waren entweder nicht infiziert, infiziert mit dem rekombinanten Virus MC96.73 oder mit der ie1-Mutanten MM96.01 in Gegenwart von Cycloheximid (50 µg/ml) für 3 Stunden, um eine selektive Expression der immediate-early Proteine zu erzielen (16). Nach Entfernung des Cycloheximids wurde Actinomycin D (5 µg/ml) hinzugefügt und die Proteine wurden mit ³⁵S-Methionin (1200 ci/mmol) für zusätzliche 3 Stunden markiert. Die Lyse der Zellen und Immunopräzipitationen wurden wie beschrieben durchgeführt (28) unter Verwendung von Antiserum 3/1, das gegen den C-Termius des ie1-Proteins pp89 gerichtet war (a) und des ie1/ie3-spezifischen Antipeptidserums b5-1 (b) (16,28). Eine lange Belichtung des Autoradiogramms (b) ist in (c) dargestellt.
Figur 6 zeigt eine Strategie zur Rekonstitution des kompletten MCMV-Genoms in *E. coli* und Exzision der Vektorsequenzen nach Transfektion des MCMV-BAC-Vektors in permissive Zellen, wie im Beispiel 3 beschrieben.
Figur 7 zeigt die Restriktionsenzymanalyse der künstlichen bakteriellen Chromosome (BACs) pSM3 und pSM3FR. Die aus *E. coli*-Kulturen isolierte BACs wurden mit EcoRI verdaut und die resultierenden DNA-Fragmente durch Elektrophorese auf Agarosegel aufgetrennt. Die EcoRI-O- und die EcoRI-Z-Banden sind angegeben und die Größe der in pSM3FR fehlenden Bande wird links gezeigt.
Figur 8 zeigt die Restriktionsenzymanalyse der viralen MW97.01- und Wildtyp-MCMV-Genome. Der BAC-Vektor pSM3FR wurde in permissive embryonale Fibroblasten (MEF) eingebracht. Nach mehreren Passagen wurde die virale DNA aus den infizierten Zellen des Stamms MW97.01 isoliert und mit EcoRI verdaut. Die resultierenden DNA-Fragmente wurden durch Elektrophorese in Agarosegelen aufgetrennt. Als Vergleich dient das mit EcoRI verdaute Wildtyp-MCMV-Genom.
Figur 9 zeigt die Überlebensraten von neugeborenen Mäusen, die mit MW97.01 bzw. MCMV wt Smith infiziert wurden.
Figur 10 zeigt die Strategie zur Klonierung des Genoms des murinen Gammaherpesvirus 68 als infektiöses bakterielles künstliches Chromosom (BAC) in *E. coli,* wie im Beispiel 4 beschrieben.
Figur 11 zeigt die Restriktionsenzymanalyse des MHV 68-BAC-Vektors und des rekonstituierten MHV 68-Genoms. Der BAC-Vektor und das aus infizierten Zellen isolierte virale Genom wurden mit EcoRI verdaut und in Agarosegel aufgetrennt. Als Vergleich dient das mit EcoRI verdaute Wildtyp-MHV 68-Genom.
Figur 12 zeigt einen Vergleich der Wachstumskinetik von MHV-Wildtyp-Virus und MHV 68-BAC-Virus. BHK-21-Zellen wurden mit einer moi von 0,01 infiziert und der Anstieg des Virus-Titers während der folgenden 4 Tage bestimmt.
Figur 13 zeigt die Klonierung des Genoms des HCMV-Laborstamms AD169 als infektiöses bakterielles künstliches Cromosom (BAC).
Figur 14 zeigt zwei unterschiedliche isomere Formen des HCMV-BAC-Genoms, pHB5 und pHB8.
Figur 15 zeigt die Restriktionsenzymanalyse der BAC-Vektoren pHB5 und pHB8. Beide Vektoren wurden mit HindIII verdaut und in Agarosegel aufgetrennt.
Figur 16 zeigt die Restriktionsenzymanalyse der Wildtyp-HCMV- und pHB5-Genome. Die virale DNA wurde aus infizierten Zellen isoliert und mit EcoRI verdaut. Anschließend wurden die resultierenden DNA-Fragmente in Agarosegel aufgetrennt. Der Pfeil zeigt die zusätzliche 2 kb-EcoRI-Bande im pHB5-Genom.
Figur 17 zeigt die in Beispiel 6 beschriebene Strategie zur Mutagenese des HCMV-BAC-Vektors pHB5.
Figur 18 zeigt den Shuttle-Vektor pST76K_SacB, ein Derivat des pST76K-Vektors.
Figur 19 zeigt eine Strategie zur Mutagenese des CMV-BAC-Vektors in *E. coli.*
Figur 20 zeigt die Restriktionsenzymanalyse der rekombinanten Vektoren pHB5, pHB 98/1 und pHB/FLP. Die isolierte DNA wurde mit EcoRi verdaut und in Agarosegel aufgetrennt.
Figur 21 zeigt die in Beispiel 7 beschriebene Strategie zur direkten Transposon-Mutagenese des infektiösen MCMV-BAC-Vektors. (a) zeigt den pST76A-TnMax8-Vektor, der durch Ligation des mit Pstl linearisierten pST76-A-Vektors mit dem pTnMax8 entsteht. (b) zeigt schematisch die Induktion der Transposition in dem pST76A-TnMaxB-Vektor.
Figur 22 zeigt die Restriktionsstelle des Transposons in dem MCMV-Kn5-Klon.
Figur 23 zeigt in (a) eine Restriktionsenzymanalyse der Wildtyp-MCMV- und MCMV-Kn5-Genome. Die aus den infizierten Zellen isolierte DNA wurde mit HindIII verdaut und in Agarosegel aufgetrennt. (b) zeigt die Ansequenzierung des Klons MCMV-Kn5 mit Primem, die an den Enden des Transposons binden. Die erhaltene Sequenz wird der MCMV-Sequenz gegenübergestellt.
Figur 24 eine Restriktionsenzymanalyse verschiedener Toledo-BAC-Klone. Die Toledo-BAC-Vektoren wurde mit EcoRI verdaut und in Agarosegel aufgetrennt. Als Vergleich dient das mit EcoRl verdaute Toledo-Wildtyp-Genom.
Figur 25 zeigt eine Restriktionsenzymanalyse verschiedener Toledo-BAC-Klone mit zwei unterschiedlichen Restriktionsenzymen. Die ersten fünf Spuren links zeigen das Hindlll-Spaltmuster des aus den verschiedenen Klonen isolierten Toledo-BAC-Vektors, die letzten fünf Spuren (rechts) das Xbal-Spaltmuster.

Im folgenden wird die Erfindung im Hinblick auf die Beispiele und Figuren im einzelnen beschrieben.

### Beispiel 1: Klonierung von MCMV-Genomsequenzen als infektiöses bakterielles künstliches Chromosom (BAC) in E. coli

### Herstellung der Viren und Zellen

Die Vermehrung von MCMV (Stamm Smith, ATCC VR-194, ATCC, Rockville, Md.) in BALB/c embyonalen Maus-Fibroblasten (MEF-) und NIH3T3-Fibroblasten; (ATCC CRL1658) wurde bereits früher beschrieben (14,15). Rekombinante Viren wurden entsprechend veröffentlichten Arbeitsvorschriften erzeugt (8, 9, 15). Um die Virusnachkommenschaft aus künstlichen bakteriellen Chromosomen (BACs) herzustellen, wurden BACs (ca. 0,5-1 µg) in MEF transfiziert, indem das Calciumphosphat-Präzipitationsverfahren verwendet wurde, wie beschrieben in (15, 16).

### Isolierung viraler DNA und von BACs

Plasmidklonierungen wurden nach Standardverfahren durchgeführt (20). Restriktionsenzyme wurden bei New England Biolabs (Bevety, MA) gekauft. Wildtyp-MCMV-DNA wurde aus Virionen hergestellt und Gesamt-Zell-DNA wurde aus infizierten Zellen isoliert, wie bereits früher beschrieben (14, 17). Zirkuläre Virus-DNA wurde durch das Verfahren von Hirt (18) isoliert und in elektrokompetente *E.* co*li*-DH10B-Stämme elektroporiert (19). BACs wurden aus *E*. *coli*-Kulturen unter Verwendung eines alkalischen Lyseverfahrens isoliert (20) und durch Präzipitation mit Polyethylenglycol gereinigt (20).

### Plasmide und Mutagenese

Für die Konstruktion der Rekombinationsplasmide pRP2 und pRP3 wurde ein 17 kb-HindIII-BamHI-Subfragment des MCMV-HindIII-E'-Fragments (17) in pACYC177 (21) subkloniert. Die EcoRI-Fragmente O, b, f und g innerhalb des HindIII-E'-Fragments (14, siehe Figur 2), wurden deletiert und ein EcoRI-Notl-Adapter wurde hinzugefügt, um das Plasmid pHE'△E zu erzeugen. Das *E. coli-*Guaninphosphoribosyltransferase(gpt)-Gen, gesteuert von dem Herpes simplex - Thymidinkinase-Promotor und gefolgt von der SV40 frühen Polyadenylierungsstelle wurde auf einer Seite mit einer Tandem-loxP-Stelle flankiert und dann einschließlich der Tanden-loxP site als Nsil-BamHI-Fragment in das Plasmid pKSO, einem Derivat des BAC-Vektors pBAC108L (19) mit modifiziertem Polylinker (Pmet-Nsil-Pacl-BamHl-Pmel-Ascl), kloniert. Das entstandene Plasmid pKSO-gpt wurde dann zwischen den loxP-Stellen mit einem 540 bp großen Haelll-Fragment aus pBR322 (Nukleotide 1928 - 2488) versehen, um später eine Mobilisierung des MCMV-BAC-Plasmids über das pBR322 mob-Element zu ermöglichen. Schließlich wurde das resultierende Plasmid IIBAC mit Notl neben einer der beiden IoxP-Stellen linearisiert und in die einzige Notl-Stelle des Plasmids pHE'ΔE inseriert. pRP2 und pRP3 unterscheiden sich in der Orientierung der IIBAC-Sequenz (Figur 2).

Um das Mutageneseplasmid pMieFS zu konstruieren, wurde ein 7,2 kb Hpal-EcoRI-Fragment (Figur 4a) des Plasmids plE111 (23) in das Plasmid pMBO96 (24) inseriert, und die Hindlll-Stelle des Inserts wurde durch Behandlung mit der Klenow-Polymerase aufgefüllt und zerstört. Die Mutagenese des MCMV-BACs wurde durch homologe Rekombination in dem *E*. *coli*-Stamm CBTS (25) entsprechend veröffentlichten Arbeitsvorschriften (24, 25) durchgeführt.

### Konstruktion rekombinanter MCMV

Rekombinante Viren wurden entweder durch Kotransfektion von viraler DNA und dem linearisierten Rekombinationsplasmid, wie vorher beschrieben (8, 15), oder durch Elektroporation des Rekombinationsplasmids in NIH3T3-Zellen unter Verwendung des BioRad Genpulsers (250 V, 960 µF), gefolgt von einer Superinfektion mit MCMV 8 Stunden später, erzeugt. Rekombinante Viren wurden mit Mycophenolsäure und Xanthin entsprechend veröffentlichter Arbeitsvorschriften (9) selektiert.

### Isolierung viraler DNA

Wildtyp-MCMV-DNA wurde aus Virionen hergestellt und durch CsCI-Gradientenzentrifugation gereinigt, wie vorher beschrieben (14). Für eine Charakterisierung der rekonstituierten Genome wurde die Gesamtzell-DNA aus infizierten Zellen isoliert. Die Zellen wurden durch Trypsinierung geerntet, gefolgt von einer Zentrifugation bei 800 g für 5 Minuten und in 50 mM Tris-HCl, pH 8, 20 mM EDTA, 0,5 % SDS und 0,5 mg/ml Proteinase K lysiert. Nach 12-stündiger Inkubation bei 56°C wurde die DNA zweimal mit Phenol/Chlorofom/Isoamylalkohol (50:48:2) extrahiert und mit Ethanol ausgefällt. Die DNA-Fragmente wurden durch Elektrophorese auf einem 0,6 % Agarosegel aufgetrennt, wie vorher beschrieben (14).

Zirkuläre Virus-DNA wurde durch das Verfahren von Hirt (18) isoliert. Infizierte Zellen aus 60 mm Gewebekulturschale wurden in 1 ml Puffer A lysiert (0,6 % SDS, 10 mM EDTA pH 7,5) und 0,66 ml 5 M NaCl wurden hinzugefügt, gefolgt von einer Inkubation bei 4°C für 24 Stunden. Die Probe wurde bei 15000 g bei 4°C 30 Minuten zentrifugiert, der Überstand wurde mit Phenol extrahiert und die DNA wurde mit Ethanol ausgefällt. Die DNA wurde wieder in 30 µl TE gelöst (10 mM Tris-HCI pH 8, 1 mM EDTA) und gegen TE dialysiert. 10 µl der DNA wurden in elektrokompetente *E*. *coli*-DH10B elektroporiert, unter Verwendung eines BioRad Genpulsers (2,5 kV, 25 µF, 400 Ohm). Die Transformanten wurden auf Agarplatten selektiert, die 12,5 µg/ml Chloramphenicol enthielten.

### Isolierung von BACs und Rekonstitution von rekombinanten Viren

BACs wurden aus 100 ml Kulturen isoliert, die über Nacht bei 37°C in Gegenwart von 12,5 µg/ml Chloramphenicol angezüchtet worden waren, unter Verwendung des alkaüschen Lyseverfahrens (20). Die Plasmid-DNA wurde weiter durch Präzipitation mit Polyethylenglycol gereinigt (20). Ein Zehntel der isolierten Plasmide (ungefähr 0,5 bis 1 µg) wurde zur Analyse durch Restriktionsenzymspaltung oder für eine Rekonstitution der Virusnachkommenschaft durch Calciumphosphattransfektion in MEF verwendet.

### Metabolische Markierung und Immunopräzipitation

Eine selektive Expression von wt oder mutiertem MCMV immediate-early Proteinen wurde durch Infektion von MEF in Gegenwart von Cycloheximid (50 µg/ml) erreicht, das 3 Stunden später durch Actinomycin D (5 µg/ml) ersetzt wurde. Dann wurden Zellen mit [35S]-Methionin (1200 Ci/mmol; Amersham, Braunschweig, Deutschland) für zusätzliche 3 Stunden markiert. Die Zellen wurden lysiert und immunpräzipitationen wurden unter Verwendung des Antipeptidserums b5/1 (28, 16) und des Antiserums 3/1, das gegen den C-Terminus des ie1-Proteins pp89 gerichtet ist, durchgeführt.

### Strategie zur Klonierung und Mutagenese des MCMV-Genoms

Die bekannten Verfahren zur Manipulation von CMV-Genomen und anderen großen Virusgenome sind nur begrenzt anwendbar bzw. erfolgreich, da sie auf homologer Rekombination in eukaryontischen Zellen basieren. Um das MCMV-Genom und andere große Virusgenome einer Mutagenese besser zugänglich zu machen, wurden infektiöse künstliche bakterielle Chromosomen (BAC) des MCMV in *E. coli* erzeugt. Die in Figur 1a dargestellte Strategie wurde zur Klonierung des MCMV-Genoms verwendet, da Herpesviren ihr Genom nach dem Eintritt in die Zelle zirkularisieren (6, 26) und da plasmidähnliche zirkuläre Intermediate früh im Replikationszyklus von Herpesviren auftreten (27). In einem ersten Schritt wurde ein rekombinantes Virus erzeugt, das einen bakteriellen Vektor integriert in seinem Genom enthielt. Nach Selektion des rekombinanten Virus unter Verwendung des Selektionsmarkers Guaninphosphoribosyltransferase(gpt) (9) (auch andere Selektionsmarker, wie z.B. das Neomycin-, Hygromycin- oder Puromycin-Resistenzgen, sind ebenfalls dafür geeignet) häufen sich zirkuläre Intermediate in infizierten Zellen an. Nach Isolierung und Elektroporation der zirkulären Intermediate in *E. coli* ist das CMV-BAC der gesamten Gentechnologie zugänglich, die in Verbindung mit *E. coli* verwendet wird. Die Transfektion des mutierten BACs in eukaryontische Zellen sollte schließlich virale Mutanten rekonstituieren (Figur 1).

### Erzeugung rekombinanter Viren und BACs

Wir konnten bereits früher zeigen, daß ein großer Bereich am rechten terminalen Ende des MCMV-Genoms nicht essentiell für die Replikation in vitro ist (18). Daher wurde dieser Bereich für eine Integration des BAC-Vektors und des Selektionsmarkers gpt (Figur 2) gewählt. Um zu untersuchen, ob die Integration des BAC-Vektors in das virale Genom in beiden Orientierungen möglich war, wurden zwei unterschiedliche Rekombinationsplasmide pRP2 und pRP3 hergestellt (Figur 2). Für die Herstellung des BAC-haltigen Virusgenoms wurde das rekombinante Virus ΔMC95.21 verwendet, das eine lacZ-Insertion im EcoRI-O-Fragment seines Genoms aufweist. Dies ermöglichte die Identifizierung von Integrationsereignissen, indem ein Screening auf weiße Plaques nach Färbung mit 5-Brom-3-chlorindolyl-β-galactopyranosid (X-Gal) durchgeführt wurde. Rekombinante Viren mit integrierten Vektorsequenzen wurden unter Verwendung des gpt-Markers angereichert. Schließlich wurde eine zirkuläre Virus-DNA aus infizierten Zellen isoliert und in *E. coli* elektroporiert.

Eine hohe Prozentzahl bakterieller Klone (ca. 80%) enthielten die erwarteten vollständigen Plasmide. Im Vergleich mit aus MCMV-Virions isolierter DNA enthielten die BACs pSM3 und pSM4 zusätzliche EcoRI-Fragmente mit einer Länge von 22,9 bzw. 24,3 kb (Figur 3a), abhängig von der Orientierung des integrierten Vektors (Figur 2). Die zusätzlichen Banden ergaben sich aus der Fusion der terminalen EcoRI-Fragmente, was die zirkuläre Natur der BACs (Figur 2) anzeigte; wie erwartet fehlt das 5,7 kb-EcoRI-O-Fragment in den BACs (siehe Figur 2 und 3a), und die Vektorsequenzen führten zu einer Doppelbande bei 6,4 kb (als v in Figur 3a bezeichnet). Bei dem BAC pSM3 war das 2,5 kb-EcoRI Z-Fragment um 1,4 kb der gpt- und Vektorsequenzen vergrößert, was zu einem 3,85 kb-Fragment führte (Figur 3, Bande pSM3). Eine Southern-Blot-Analyse und Charakterisierung der BACs mit Restriktionsenzymen HindIII, Xbal und BamHI (Daten nicht dargestellt) bestätigten die erfolgreiche Klonierung des gesamten Genoms dieser MCMV-Rekombinanten in *E. coli.*

### Rekonstituierung (Wiederherstellung) der Virusnachkommenschaft aus MCMV-BACs

Die Transfektion der BACs pSM3 und pSM4 in embryonale Maus-Fibroblasten führten zu der Entwicklung von Plaques. Neue Zellen wurden mit dem Überstand von pSM3 und pSM4 transfizierten Zellen infiziert. Gesamtzell-DNA wurde isoliert, als die Zellen einen vollständigen cytopathischen Effekt zeigten. Die EcoRI-Spaltung der isolierten DNA führte zu einem ähnlichen Muster wie die Spaltung der BACs pSM3 und pSM4 (vgl. Banden MC96.73 und MC96.74 in Figur 3b und Banden pSM3 und pSM4 in Figur 3a). DNA, die aus infizierten Zellen isoliert wird, enthält zirkuläre, konkatemäre und lineare virale DNA, die bereits in Kapside verpackt war. Daher war die Menge der 22,9 und 24,3 kb-Fragmente, die sich aus der Fusion der terminalen EcoRI-Fragmente ergab, submolar (Figur 3b, Banden MC96.73 und MC96.74). Außerdem tauchten die terminalen EcoRI-Fragmente, die von linearen Genomen abstammten, wieder auf. Das terminale EcoRI-Fragment F (12,3 kb) wurde in dem Genom der Rekombinanten MC96.73 wie auch im Wildtyp MCMV entdeckt (Figur 3c, Spuren MC96.73 und wt). Bei der Rekombinanten MC96.74 war das terminale EcoRI-Fragment F um eine 1,4 kb-Vektorsequenz (siehe Figur 2b) vergrößert, was zu einer Doppelbande bei 13,7 kb führte (Figur 3c, Spur MC96.74). Verdau mit den Restriktionsenzymen BamHl und Xbal ergab alle erwarteten Banden (Daten nicht dargestellt). Damit wurde erreicht, daß CMV-Rekombinanten aus einem großen Plasmid ohne irgendeine Manipulation vor der Transfektion hergestellt werden können.

### Beispiel 2: Konstruktion einer MCMV-ie1-Mutanten durch homologe Rekombination in E. coli

Um die Wirksamkeit der gezielten Mutagenese in *E. coli* zu überprüfen, wurde eine kleine Mutation in die (immediate-early) (IE) Region des MCMV-Genoms eingeführt. Mindestens zwei alternativ gespleißte Transkripte ergeben sich aus der IE-Region (Figur 4a), die das 89 kDa-ie1-Protein und das 88 kDa-ie3-Protein kodieren (16). Aufgrund der komplexen Struktur der ie1/ie3-Transkriptionseinheit ist die Unterbrechung des ie1-Gens durch konventionelle Rekombinationsverfahrens vermutlich schwierig zu erreichen, ohne daß die Expression des ie3-Gens beeinflußt wird. Daher war nicht bekannt, ob das MCMV-ie1-Protein für die Virusreplikation essentiell ist. Um den ie1-Leserahmen (595 Kodons) zu unterbrechen, wurde eine Leserahmen-Mutation an einer HindIII-Stelle in Exon 4 des ie1-Gens eingeführt. Die Mutation beendete den ursprünglichen Leserahmen nach Kodon 273 und erzeugte ein neues Stoppkodon nach zusätzlichen 9 Kodons (Figur 4a). Die Mutation wurde auf einem 7,4 kb-EcoRI-HpaI-Fragment (Figur 1a) konstruiert und anschließend auf das BAC pSM3 durch homologe Rekombination in *E. coli* unter Verwendung einer zweistufigen Austauschstrategie übertragen (24, 25). Das Mutageneseverfahren führte zu einem Verlust der Hindlll-K- und -L-Fragmente und der Erzeugung eines neuen 15,2 kb-Fragments (Figur 4b). Die EcoRI- und BamHI-Muster der BACs waren unverändert (Daten nicht dargestellt), was bestätigte, daß die BACs während des Mutageneseverfahrens stabil blieben. Die Transfektion des mutierten BACs pSM4-ie in MEF führte zu Plaquebildung. Gesamzell-DNA wurde aus infizierten Zellen isoliert und durch Hind-III-Verdauung analysiert. Wie erwartet, waren die Hindlll-K- und -L-Fragmente durch das 15,2 kb-Fragment im Genom des ie1-Mutantenvirus MM96.01 ersetzt worden (Figur 4c). Offensichtlich wurde die Mutation, die in das MCMV-BAC eingeführt worden war, nach Rekonstitution des Mutantenvirus beibehalten.

Die Abwesenheit des ie1-Proteins in infizierten MM96.01-Zellen wurde durch Immunpräzipitation bestätigt. Ein Antiserum, das gegen den Carboxyterminus des ie1-Proteins gerichtet war, spürte das ie1-Protein in Lysaten von MC96.73 infizierten Zellen auf, präzipitierte aber keine Proteine aus Lysaten von MM96.01 infizierten Zellen (Figur 5a). Ein ie1/ie3-spezifisches Antiserum, B5/1, (16, 28) spürte 2 Proteine von 89 und 88 kDa in Lysaten von MC96.73 infizierten Zellen auf und ein Protein von 88 kDa in Lysaten von MM96.01 infizierten Zellen (Figur 5b). In der MM96.01-Spur fehlte das 89 kDa-ie1-Protein eindeutig und es wurde nur das 88 kDa-ie3-Protein nachgewiesen (Figur 5b). Eine längere Belichtung des Autoradiogramms zeigte ein 36 kDa-Protein in MM96.01 infizierten Zellen (Figur 5c). Das anscheinende Molekulargewicht dieses Polypeptids stimmt mit der erwarteten Molekularmasse des verkürzten ie1-Proteins und mit dem Wanderungsverhalten verschiedener mutierter ie1-Proteine überein (28). Wir schlossen daraus, daß das ie1-Protein pp89 für eine Replikation des ie-Mutanten MM96.01 nicht notwendig ist.

### Mutageneseverfahren

Folgend den veröffentlichten Arbeitsvorschriften von O'Connor et al. (24) und Kempkes et al. (25) wurde die Mutagenese des MCMV-BACs durch homologe Rekombination in dem *E. coli*-Stamm CBTS durchgeführt, der ein recA-Allel und einen temperaturempfindlichen Supressor trägt (25). Das BAC-pSM3 und das Shuttleplasmid pMieFS wurden aufeinanderfolgend in *E. coli-*CBTS elektroporiert und Klone, die Kointegrate enthielten, wurden bei 42°C auf Agarplatten mit Chloramphenicol (12,5 µg/ml) und Tetracyclin (10 µg/ml) selektiert. Die Kointegrate wurden durch Restriktionsenzymanalyse auf die gewünschten Strukturen untersucht. Die Auflösung der Kointegrate wurde durch Inkubation der bakteriellen Klone bei 30°C auf Agarplatten nur mit Chloramphenicol ermöglicht. Die Plasmide wurden durch Screening von Tetracyclin-empfindlichen Klonen identifiziert und durch HindIII-Spaltung analysiert, um zu bestimmen, ob eine Mutation erreicht worden war, oder ob eine Rückkehr zu der Eiternsequenz aufgetreten war.

### Beispiel 3: Rekonstitution des kompletten MCMV-Genoms in E. coli (Beispiel für Insertion) und Exzision der Vektorsequenzen nach Transfektion der BAC-Plasmide in permissive Zellen.

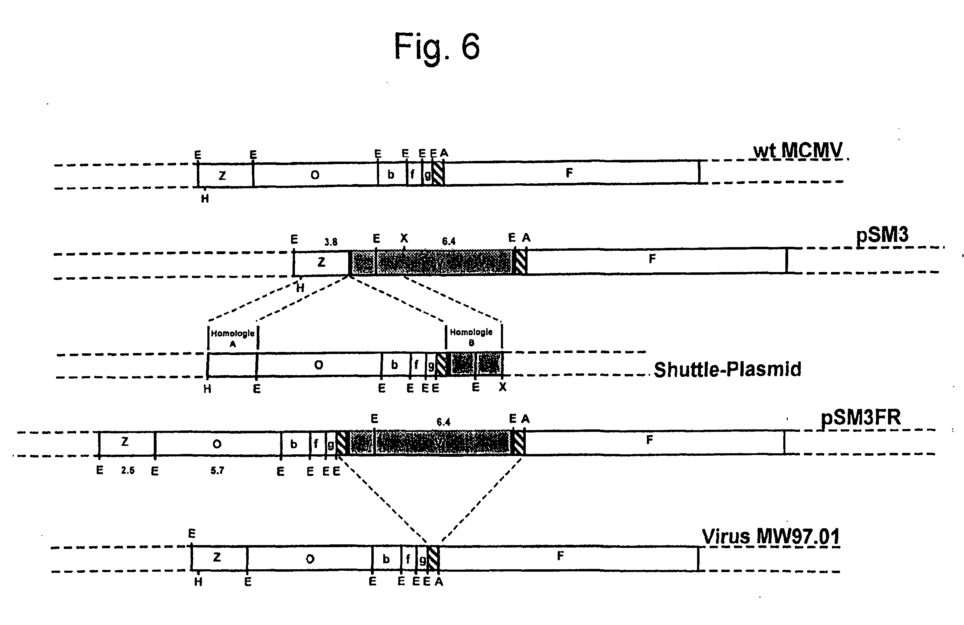

Dem MCMV BAC-Plasmid pSM3 (siehe Beispiel 1) fehlen konstruktionsbedingt 8 kb des MCMV-Genoms. Um MCMV-Mutanten generieren zu können, die auf dem vollständigen MCMV-Genom basieren, wurde ein BAC-Plasmid konstruiert, das das vollständige MCMV-Genom enthält. Dazu wurden durch homologe Rekombination in *E. coli* die fehlenden 8 kb in pSM3 eingefügt. Das resultierende Plasmid erhielt den Namen pSM3FR (vgl. Figur 6).

Zur Konstruktion des Shuttle-Plasmids wurde ein 10,9 kb HindIII-AvrII-Fragment aus dem HindIII E'-Fragment des MCMV-Stamms K181 (17) unter Verwendung eines oligonukleotid-Adapters mit den Spaltstellen XbaI-Notl-BamHI-SpeI-HindIII-BamHI in das Plasmid pACYC184 (21) kloniert. Anschließend wurde ein 2.0 kb Notl-Xbal-Fragment (Homologie B in Fig. 6) aus dem Plasmid KSO-gpt (vgl. Beispiel 1) neben dem 10.9 kb HindIII-Avril-Fragment inseriert (unter Verwendung der Spaltstellen NotI und Spel im Bereich des Oligonukleotids; vgl. Fig. 6 Mitte "Shuttle-Plasmid"). Das vollständige Insert wurde dann als 12.9 kb HindIII-Fragment in das HindIII geschnittene Plasmid pMBO96 (24) ligiert. Die Mutagenese wurde wie in Beispiel 1 beschrieben durchgeführt.

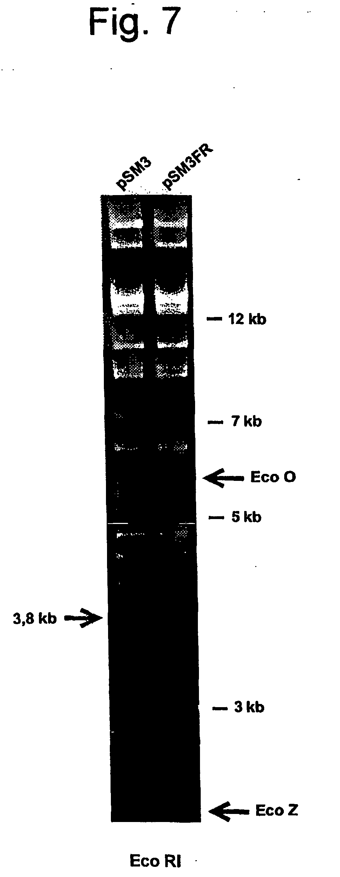

Figur 7 zeigt die EcoRI-Spaltmuster der MCMV-BAC-Plasmide pSM3 und pSM3FR. Im Plasmid pSM3FR fehlt im Vergleich zu Plasmid pSM3 wie erwartet eine 3.8 kb EcoRI-Bande, und die 5.7 kb EcoRI O- und die 2.5 kb EcoRI Z-Banden sind wie im MCMV wt-Genom wieder vorhanden (vgl. dazu die schematische Darstellung der erwarteten Struktur der BAC-Plasmide pSM3 und pSM3FR in Fig. 6). Die EcoRI-Fragmente b, f und g sind ebenfalls in pSM3FR vorhanden (sie haben wegen ihrer geringen Größe eine größere Mobilität in Agarose-Gelen und sind in dem in Fig. 7 abgebildeten Gelausschnitt nicht enthalten).

Das BAC-Plasmid pSM3FR enthält also das gesamte MCMV-Genom und den BAC-Vektor pKSO-gpt flankiert von loxP-Stellen (schwarze Bereiche in Fig. 6).

Bei der Insertion der fehlenden 8 kb wurde gleichzeitig ein 527 bp großes EcoRI-AvrII-Fragment links von den Vektorsequenzen miteingefügt, das rechts von den Vektorsequenzen schon vorhanden ist (schraffiert in Fig. 6). Dieser Bereich ist im BAC-Plasmid pSM3FR also doppelt vorhanden und flankiert die BAC-Vektorsequenzen (grau markiert in Fig. 6).

Identische Sequenzen sind Voraussetzung für homologe Rekombination. Die Flankierung der BAC-Vektorsequenzen mit dem 527 bp Fragment wurde mit dem Ziel vorgenommen, nach Transfektion des BAC-Plasmids pSM3FR in permissive, eukaryonte Zellen die Vektorsequenzen durch homologe Rekombination herauszuschneiden. Im *E. coli*-Stamm CBTS (25) ist das BAC-Plasmid pSM3FR stabil, da in diesem *E. coli*-Stamm wesentlich größere homologe Bereiche benötigt werden, um eine homologe Rekombination herbeizuführen.

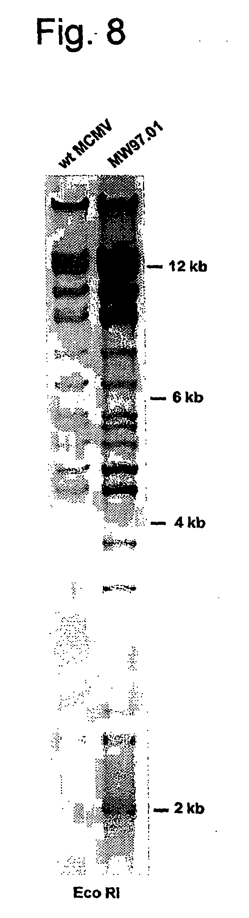

Nach Transfektion des BAC-Plasmids pSM3FR in permissive embryonale Fibroblasten (MEF) wurde eine spontane Exzision der Vektorsequenzen aus dem MCMV-BAC-Genom durch homologe Rekombination über die 527 bp Sequenzen beobachtet. Nach wenigen Passagen des rekonstituierten Virus in diesen Zellen sind die Vektorsequenzen nicht mehr nachweisbar (weder durch Southern Blotting noch durch PCR). Der Vergleich des EcoRI-Spaltmusters des Wildtyp-MCMV-Genoms mit dem des MW97.01-Genoms (das aus dem BAC-Plasmid pSM3FR regeneriert wurde), läßt keine Unterschiede erkennen (Fig. 8). Da die eingefügten 8 kb dem Genom des MCMV-Stamms K181 entstammen und geringe Sequenzunterschiede (Polymorphismus) zu dem verwendeten Widtyp-MCMV-Stamm Smith aufweisen, können bzw. konnten wir jedoch eindeutig nachweisen, daß sich das MW97.01-Virusgenom von dem BAC-Plasmid pSM3FR ableitet.

Offensichtlich werden MCMV-Genome, die die bakteriellen Vektorsequenzen verloren haben, wesentlich besser in Viruspartikel verpackt als MCMV-Genome mit Vektorsequenzen, da letztere ein überlanges Genom besitzen.

Flankierung des BAC-Vektors mit identischen Sequenzen kann also dazu verwendet werden, die Vektorsequenzen aus dem Virusgenom wieder zu entfernen. Dieses Verfahren scheint insbesondere geeignet, wenn es in Kombination mit dem Selektionsdruck gegen ein überlanges Genom verwendet wird. Das Verfahren ist eine Alternative zum Herausschneiden der Vektorsequenzen mit Rekombinase Cre (vgl. Beispiel 4).

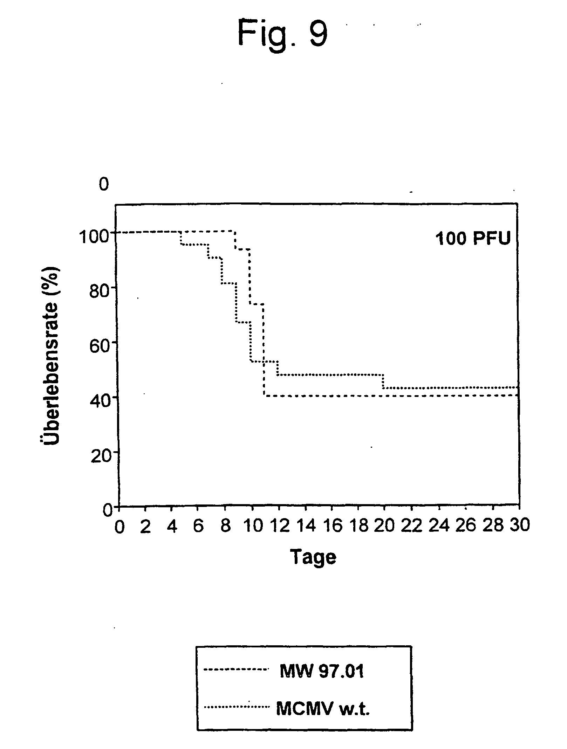

Das von dem MCMV-BAC-Plasmid pSM3FR abgeleitete Virus MW97.01 wurde bezüglich seiner Virulenz in neugeborenen Mäusen mit dem MCMV-Wildtyp-Stamm Smith verglichen. Figur 9 zeigt identische Überlebensraten von Mäusen, die mit MW97.01 bzw. MCMV wt Smith infiziert wurden. Die Virus-Titer in verschiedenen Organen waren ebenfalls vergleichbar hoch (Daten nicht gezeigt). Das Virus MW97.01 weist also die gleichen biologischen Eigenschaften wie das Wildtyp-MCMV-Virus auf. Dies zeigt, daß die Passagierung des MCMV-Genoms in *E*. *coli* nicht zu spontanen Veränderungen des MCMV-Genoms und der biologischen Eigenschaften des regenerierten Virus führt.

### Beispiel 4: Klonierung des Genoms eines Gammaherpesvirus (murines Gammaherpesvirus 68 - MHV 68 -) als infektiöses bakterielles künstliches Chromosom (BAC) in E. coli und Rekonstitution von MHV 68-Viren.

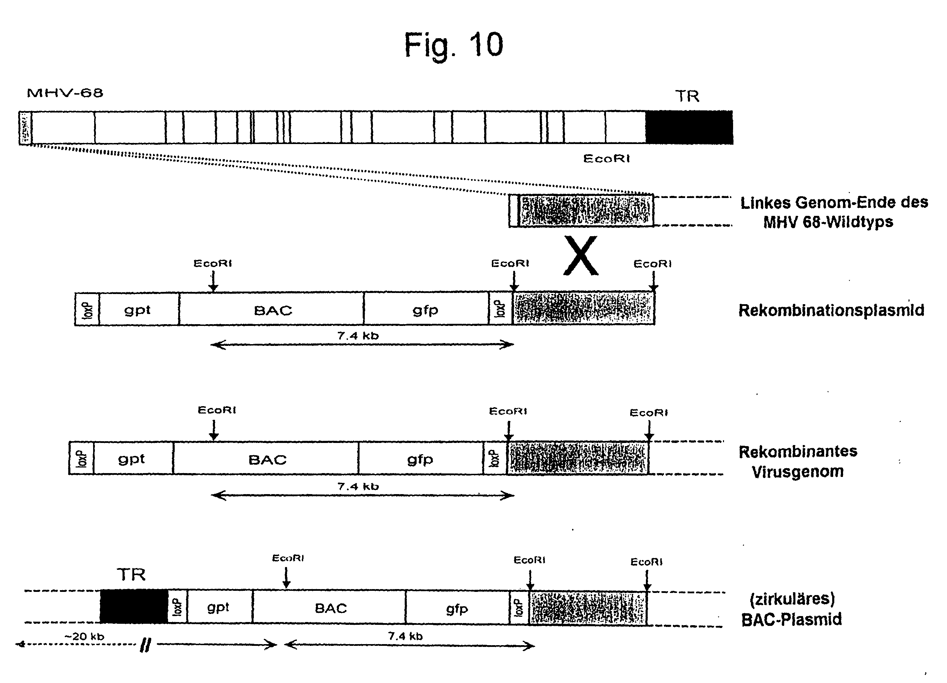

Dieses Beispiel zeigt exemplarisch die Klonierung eines Gammaherpesvirus-Genoms. Das Maus-Gammaherpesvirus 68 (MHV 68) ist ein natürliches Pathogen für Mäuse und zeigt in seiner Biologie und in seiner Genom-Organisation Ähnlichkeiten zu den humanen Herpesviren Epstein-Barr-Virus und HHV8 (29). Infektion der Maus mit MHV 68 ist deshalb ein hervorragend geeignetes Modell-System, um die Pathogenese von Gammaherpesvirus-Infektionen zu untersuchen. Das Genom des murinen Gammaherpesvirus 68 (MHV 68) enthält an seinem Ende eine wechselnde Zahl von terminalen Repeats (TR; Fig. 10 oben). Das MHV 68-Genom kann Insertionen durch eine geringere Zahl an terminalen Repeats kompensieren. Vor kurzem wurde gefunden, daß Insertionen am linken Genomende von MHV 68 durch einseitiges Crossing over mit dem linearen Virusgenom möglich sind (30). Deshalb wurden die BAC-Vektorsequenzen durch einseitiges Crossing-over am linken Ende des Genoms eingefügt.

Zur Konstruktion des Rekombinationsplasmids wurde ein 1.5 kb großes EcoRI-Fragment vom linken Ende des Genoms durch PCR mit MHV 68-DNA als template generiert und in die EcoRI-Schnittstelle des Plasmids pMHV3 kloniert (ein pK18-Derivat, dessen Polylinker zuvor durch Insertion eines Oligonukleotids modifiziert wurde (neue Spaltstellen: Mlul-Notl-Avrll-SgrAl-Pacl-SgrAl-EcoRl-ApaLl-Mlul)). (pK18 ist ein Derivat von pUC18, das anstelle des Ampicillin-Resistenz-Gens eine Kanamycin-Resistenz-Gen enthält). Dann wurde über eine Pacl-Schnittstelle links von dem EcoRI-Fragment der BAC-Vektor pKSO-gpt (vgl. Beispiel 1) eingefügt (vgl. Figur 10; 3. Zeile: "Recombinationsplasmid"). Schließlich wurde unter Verwendung eines MluI-PstI-Adapters ein 1.6 kb großes NsiI-MluI Fragment des Plasmids EGFP (Clontech), das den HCMV major immediate-early Promoter, die kodierende Sequenz für das Green fluorescent Protein (gfp) und das SV40-polyA-Signal enthält, in eine Nsil-Schnittstelle zwischen BAC-Vektorsequenzen und der rechten IoxP-Stelle kloniert (vgl. Fig. 10; "Recombinationsplasmid").

Fünf µg des Rekombinationsplasmids wurden zur Freisetzung des Inserts (loxPgpt-BAC-Vektor-gfp-loxP-homologer Bereich) mit Mlul gespalten. Das Insert wurde zusammen mit ca. 2 - 5 µg viraler MHV 68-DNA (isoliert aus MHV 68 infizierten BHK-21 Zellen) in BHK-21 Zellen elektroporiert. Rekombination zwischen den homologen Sequenzen des linearen MHV 68-Genoms und des Rekombinationsplasmids (grau in Fig. 10; 2. und 3. Zeile) sollte zur Insertion des BAC-Vektors und zur Bildung des rekombinanten Virus-Genoms führen (4. Zeile in Fig. 10). Ligation des rechten Genom-Endes (mit den terminalen Repeats) an das linke Genom-Ende (das jetzt den BAC-Vektor enthält) führt zur Bildung eines zirkulären MHV 68-BAC-Genoms, das aus infizierten Zellen isoliert, in *E*. *coli* elektroporiert und schließlich in *E*. *coli* als BAC-Plasmid propagiert werden kann.

Nach Elektroporation von viraler DNA und Rekombinationsplasmid in die BHK-21 Zellen entstanden Plaques. Nach Erreichen eines kompletten cytopathischen Effekts wurde das Virus auf neue BHK21-Zellen übertragen und einer Selektion mit Xanthine (25 µM) und Mycophenolsäure (100 µM) unterzogen (9,11). Dies sollte zur Anreicherung von Viren führen, die das BAC-Plasmid mit dem Selektionsmarker gpt in ihr Genom integriert haben. Im Fluoreszenz-Mikroskop konnten grün-gefärbte Zellen nachgewiesen werden, ein Hinweis auf die Integration und Expression des gfp-Gens. Nach drei Selektionsrunden mit Xanthine und Mycophenolsäure wurde zirkuläre MHV 68-DNA aus infizierten BHK 21-Zellen isoliert, in *E*. *coli* DH10B elektroporiert und auf Agarplatten mit Chloramphenicol (12.5 µg/ml) ausplattiert (vgl. Beispiel 1). Plasmid-Isolierung mit anschließendem Restriktionsverdau führte zur Identifizierung eines *E*. *coli*-Klons, der ein BAC-Plasmid mit vollständigem MHV 68-Genom enthält.

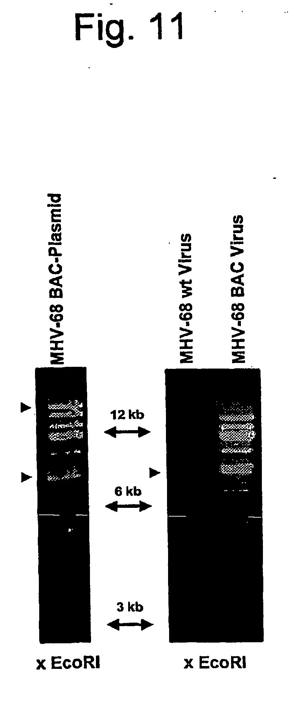

Restriktionsverdau des MHV 68-BAC-Plasmids mit EcoRI -zeigt im Vergleich zu EcoRI-gespaltener MHV 68-Wildtyp-Virus-DNA eine Doppelbande bei 7.4 kb und eine zusätzliche Bande bei ca. 20 kb (Fig. 11; vgl. Spur MHV 68-BAC-Plasmid mit Spur MHV 68-wt-Virus; Unterschiede sind mit schwarzen Pfeilspitzen markiert). Die zusätzliche 7.4 kb Bande (die zur Bildung der Doppelbande führt) entstammt dem BAC-Vektor-Anteil und die zusätzliche ca. 20 kb große Bande resultiert aus der zirkulären Natur des Plasmids (während die ca. 20 kb große Bande in Spur MHV 68-wt-Virus fehlt, da es sich bei der MHV 68-Virus-DNA überwiegend um lineare Genome handelt; vgl. schematische Darstellung des MHV 68-BAC-Plasmids in Fig. 10 unten). Restriktionsverdaus des BAC-Plasmids mit anderen Restriktionsenzymen bestätigten die erwartete Struktur des MHV 68-BAC Plasmids.

Transfektion des MHV 68-BAC-Plasmids in BHK 21-Zellen führte zur PlaqueBildung. Dies zeigt, daß das MHV 68-BAC-Plasmid "infektiös" ist, alle essentiellen Gene enthält und daß während der Propagierung des Plasmids in *E. coli* keine unerwünschten Mutationen eingetreten sind. Infizierte Zellen zeigten im Fluoreszenz-Mikroskop eine grüne Färbung, d.h. das gfp-Gen ist wie erwartet im Genom der rekonstituierten Viren enthalten. DNA des MHV 68-BAC-Virus wurde aus infizierten Zellen isoliert, mit EcoRI verdaut und in einem Agarosegel aufgetrennt (Fig. 11, Spur "MHV 68-BAC-Virus"). Das EcoRI-Spaltmuster der MHV 68-BAC-Virus-DNA ist wie erwartet im wesentlichen identisch mit dem EcoRI-Spaltmuster der MHV 68-wt-Virus-DNA (Fig. 11); die ca. 20 kb große EcoRI-Bande ist verschwunden, da die aus infizierten Zellen isolierte MHV 68-BAC-Virus-DNA ebenfalls überwiegend lineare Genome umfaßt.

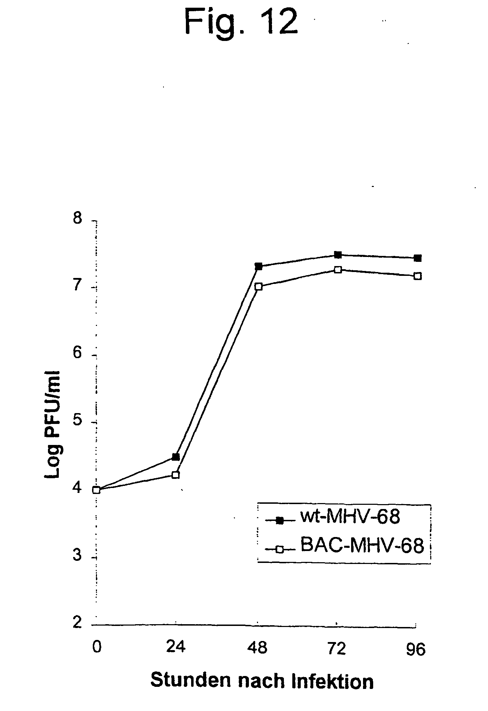

Figur 12 zeigt einen Vergleich der Wachstumskinetik von MHV-Wildtyp-Virus und MHV 68-BAC-Virus. BHK-21-Zellen wurden mit einer moi von 0,01 infiziert und der Anstieg des Virus-Titers während der folgenden 4 Tage bestimmt.

Die Wachstumskinetik der beiden Viren ist identisch; ein weiterer Nachweis, daß das MHV 68-BAC-Genom für alle Virus-Funktionen kodiert- und daß die Vermehrung des MHV 68-BAC-Genoms in *E*. *coli* möglich ist.

Die BAC-Vektorsequenzen (mit dem gpt- und dem gfp-Gen) lassen sich mit Cre-Rekombinase (22) über die loxP-Stellen aus dem MHV 68-BAC-Genom wieder entfernen (vgl. Fig. 10). Dadurch läßt sich ein MHV 68-Genom generieren, das bis auf eine loxP-Stelle identisch mit dem Wildtyp-MHV 68-Genom ist. Durch Passagierung des MHV 68-BAC-Virus in einer Zellinie, die Cre-Rekombinase exprimiert, wurden die BAC-Vektorsequenzen deletiert. Mit den resultierenden Viren infizierte Zellen zeigten keine Grün-Färbung, ein Beweis, daß die BAC-Vektorsequenzen einschließlich des gfp-Gens deletiert wurden.

### Beispiel 5: Klonierung des Genoms des HCMV-Laborstamms AD169 als infektiöses bakterielles künstliches Chromosom (BAC) und Rekonstitution von Cytomegaloviren aus transfizierten HCMV-BAC-Plasmiden

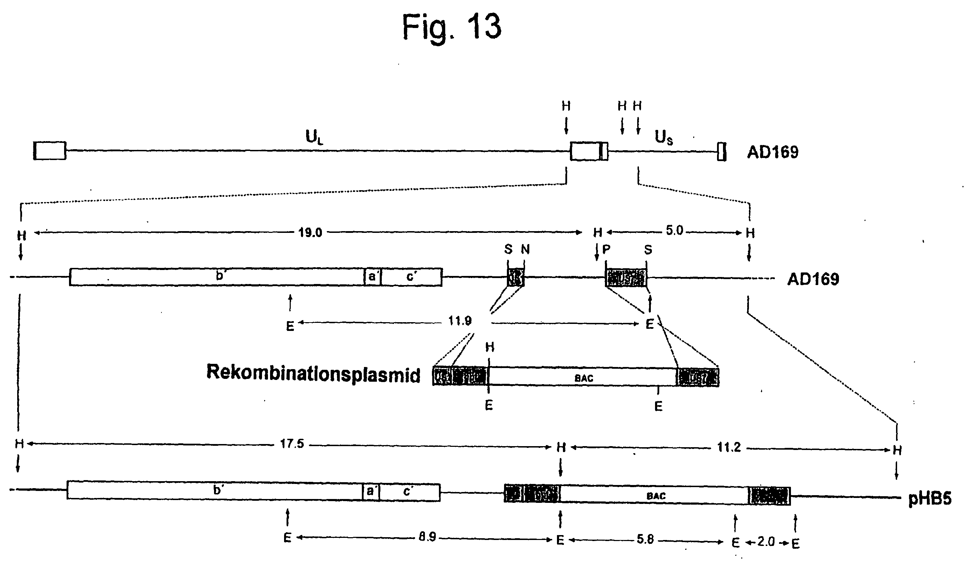

Für die Insertion des BAC-Vektors wurde der Bereich zwischen den Genen US1 und US7 in der Unique-short (Us)-Region des HCMV-Genoms ausgewählt, da bekannt war, daß dieser Bereich Gene enthält, die für die Replikation von HCMV in Zellkultur nicht essentiell sind (31). Zur Konstruktion eines Rekombinationsplasmids wurde ein 4.75 kb SacI-Fragment (Nukleotide 192648 bis 197398 des Genoms des HCMV-Laborstamms AD169; (3) in das Plasmid pGEM3Zf (Promega) kloniert. Anschließend wurde ein 2.7 kb Nhel-PshAl-Fragment innerhalb des 4.75 kb Sacl-Fragments detetiert und das gpt-Gen (unter Kontrolle des Herpes simplex virus Typ1-Thymidinkinase-Promoters und gefolgt von dem SV40-polyA-Signal; (9, 11) sowie ein Oligonukleotid-Adapter mit einer Pacl-Schnittstelle eingefügt (Fig. 13). Schließlich erfolgte die Insertion des BAC-Vektors pKSO (vgl. Beispiel 1) in die Pacl-Schnittstelle (Fig. 13). Das Rekombinationsplasmid weist an seinem linken Ende 712 bp und am rechten Ende 1355 bp an homologen Sequenzen zum HCMV-Genom im Bereich der US1- bzw. US7-Gene auf (vgl. Fig. 13 Mitte). Vor der Elektroporation wurde das Rekombinationsplasmid mit dem Enzym Xcml im Bereich des Plasmid-Rückgrates linearisiert.

Ca. 30 µg des linearisierten Rekombinationsplasmids wurden in humane Vorhaut-Fibroblasten (HFF) elektroporiert (Biorad-Elektroporator: 250 V, 960 µF). Ca. 24 Stunden nach Elektroporation wurden die HFF-Zellen mit dem HCMV-Laborstamm AD169 mit einer moi von 1 infiziert. Nach vierstündiger Adsorption des Virus wurden die Zellen gewaschen und neues Medium zugegeben. Anschließend wurden die Zellen bei 37°C und 5% CO₂ so lange weiterkultiviert, bis ein vollständiger cytopathischer Effekt eingetreten war. Der virushaltige Zellkulturüberstand wurde dann auf neue HFF-Zellen übertragen und rekombinante Viren durch Selektion mit Mycophenolsäure (100 µM) und Xanthin (25 µM) angereichert (9, 11). Bei Eintritt eines vollständigen cytopathischen Effekts wurde das Virus wiederum auf neue HFF-Zellen passagiert und einer weiteren Selektionsrunde unterzogen. Nach der 3. Selektionsrunde wurde zirkuläre Virus-DNA mit dem Hirt-Verfahren (18; vgl. Beispiel 1) aus den infizierten Zellen isoliert und in *E. coli* DH10B elektroporiert wie in Beispiel 2 beschrieben.

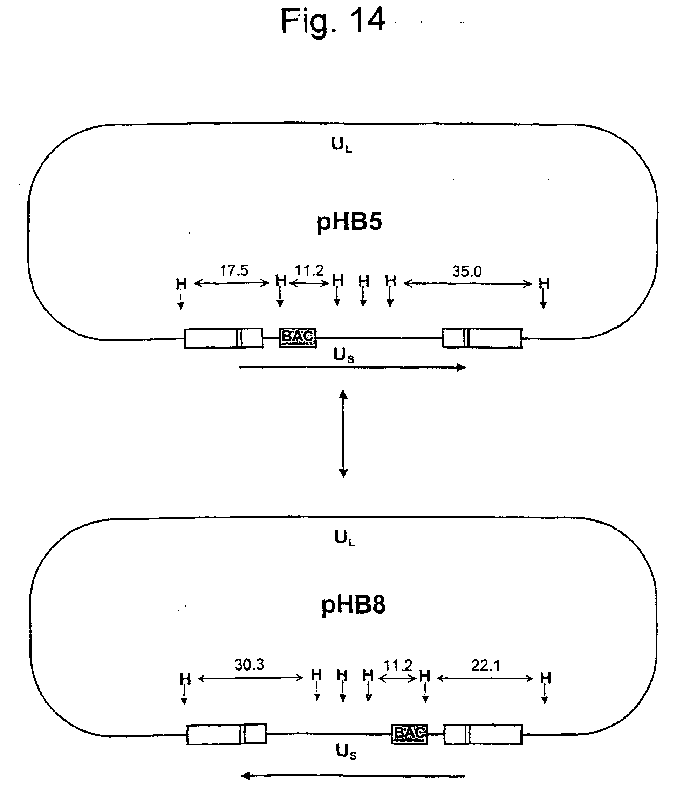

Die Unique-long (UL)-Region und die Unique-short (Us)-Region des HCMV-Genoms können ihre Orientierung durch Inversion an den internen und terminalen Repeats (offene Rechtecke in Fig. 13) relativ zueinander ändern (3). Nach Integration des BAC-Vektors an der erwarteten Position in der Us-Region (vgl. Fig. 13 unten) werden zwei unterschiedliche isomere Formen des HCMV-BAC-Genoms erwartet, die sich durch Restriktionsverdau mit Hindlll unterscheiden lassen (Fig. 14).

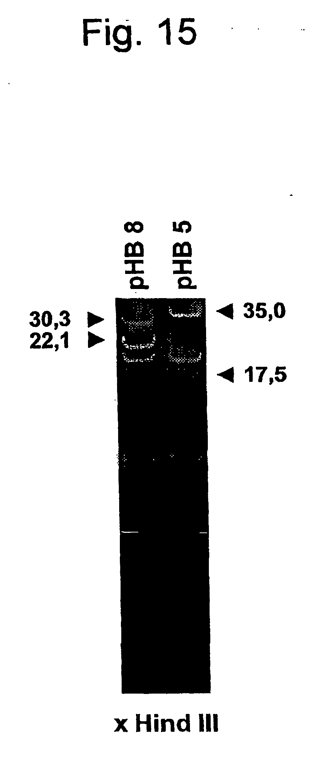

Durch Plasmidisolierung mit anschließendem HindIII-Restriktionsverdau wurden *E*. *coli*-Klone identifiziert, die BAC-Plasmide mit dem HCMV-Genom enthalten und es wurden HCMV-BAC-Plasmide mit den beiden unterschiedlichen isomeren Formen gefunden (Fig. 15). Prototypen sind die beiden BAC-Plasmide pHB5 und p_{H}B8 (Fig 15). pHB5 weist HindIII-Fragmente von 17.5 und 35 kb auf, während pHB8 mit HindIII-Fragmenten von 22.1 und 30.3 kb die andere Orientierung zeigt.

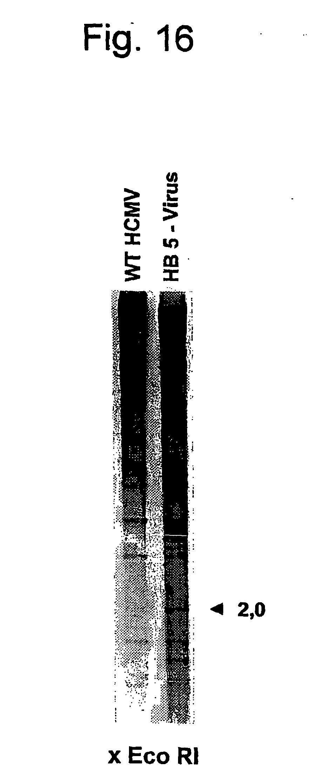

Transfektion von pHB5 und pHB8 mit Superfect (Qiagen, Hilden, Germany) in HFF-Zellen bzw. MRC5-Fibroblasten führte nach ca. 10 bis 14 Tagen zur Plaquebildung. Die HCMV BAC-Plasmide wurden zusammen mit einem Plasmid transfiziert, das das CMV-Protein pp71 exprimiert, da bekannt ist, daß pp71 die Infektiosität viraler DNA erhöht (37). Pro Kulturschale (Durchmesser 6 cm) wurden 0.5 - 3 µg HCMV-BAC-Plasmid und 1 µg des pp71-Expressionsplasmids kotransfiziert. Virale DNA wurde aus pHBS-BAC-Virus infizierten und zum Vergleich aus HCMV-Laborstamm AD169 infizierten Zellen isoliert (vgl. Beispiel 1), mit EcoRI verdaut und in 0.5%igen Agarose-Gelen aufgetrennt (Fig. 16). Dem pHB5-Genom fehlt im Vergleich zum HCMV-AD169-Genom ein 11.9 kb EcoRI-Fragment, dafür besitzt das pHB5-Genom neue EcoRI-Fragmente von 8.9, 5.8 und 2.0 kb (vgl. Fig. 13, Mitte und unten).

Die zusätzliche 2.0 kb EcoRI-Bande im pHB5-Genom ist in Figur 16 gut zu erkennen. Die zusätzlichen 5.8 und 8.9 kb-EcoRI-Banden und das Fehlen der 11.9 kb-EcoRI-Bande wurde durch Southern-Blotting bestätigt. Das pHB5-Genom weist also die erwartete Struktur auf (vgl. Fig. 16). Das pHB8-Genom wurde in ähnlicher Weise charakterisiert und zeigte ebenfalls die erwartete Struktur.

Das Genom des HCMV-Laborstamms AD169 konnte nach Insertion des BAC-Vektors zwischen den US1- und US7-Genen als BAC-Plasmid in *E. coli* kloniert werden. Es wurden beide zu erwartenden isomeren Formen gefunden. Aus beiden Konformationen konnten nach Transfektion in permissive humane Fibroblasten infektiöse Viren rekonstituiert werden.

### Beispiel 6: Mutagenese des HCMV-BAC-Plasmids pHB5 und Herstellung von HCMV-Mutanten.

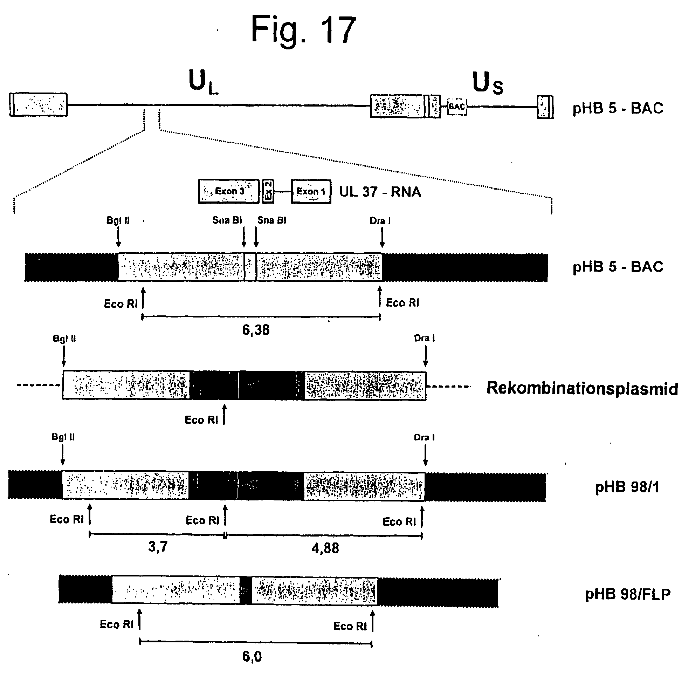

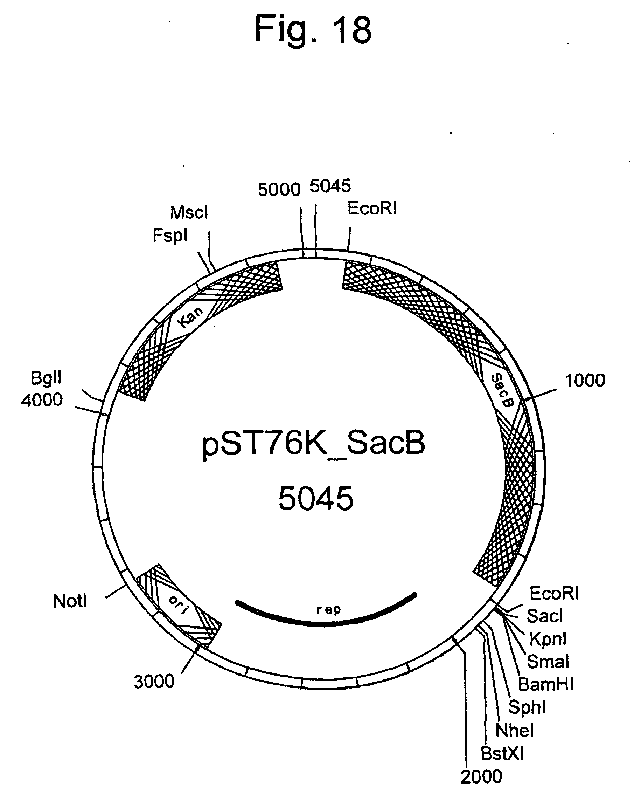

Um zu zeigen, daß sich mit dem Verfahren HCMV-Mutanten herstellen lassen, wurden 380 bp in Exon-3 des HCMV-UL37-Gens (3) deletiert und durch eine 2.6 kb Tetrazyklin-Kassette (32) ersetzt. Für die Mutagenese wurde folgendes Rekombinationsplasmid konstruiert: Ein 9.7 kb BgIII-Fragment aus dem HCMV AD169-Genom (Genompositionen 47366-57120; 33, 3) wurde in ein pBluescript-Derivat kloniert (dessen Polylinker durch Insertion eines Oligonukleotids zuvor modifiziert wurde und eine BgIII-Schnittstelle enthielt). Dann wurde ein 380 bp SnaBI-Fragment aus dem 3. Exon des UL37-Gens herausgeschnitten und eine 2.6 kb Tetrazyklin-Kassette eingefügt (vgl. Fig. 17 "Recombinationsplasmid"). Schließlich wurde das 8.9 kb BgIII-DraI-Fragment in das BamHI-SmaI-geschnittene Shuttle-Plasmid pST76K_SacB transferiert (Fig. 18). Das Shuttle-Plasmid pST76K_SacB ist ein Derivat des Plasmids pST76K (34). pST76K_SacB (Fig. 18) enthält zusätzlich zum Kanamycin-Resistenz-Gen und dem temperatur-sensitiven Replikationsmechanismus den Negativ-Selektionsmarker SacB, der eine Selektion gegen das Plasmid auf Agarplatten mit 5% Saccharose ermöglicht.

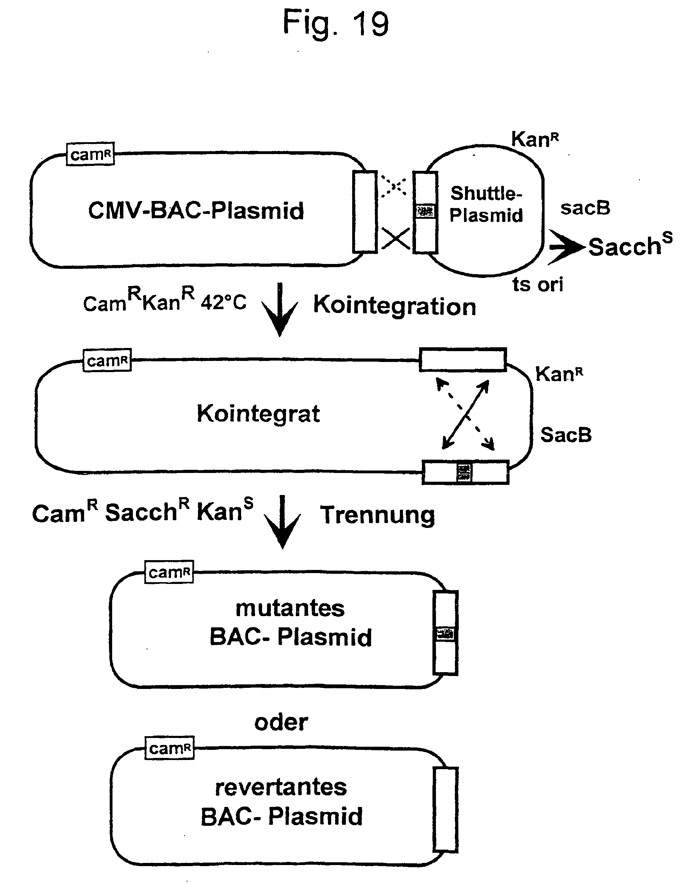

Die Mutagenese erfolgte ähnlich dem in Beispiel 2 beschriebenen Verfahren (vgl. Fig. 19). Das HCMV-BAC-Plasmid pHB5 und das Rekombinationsshuttle-Plasmid wurden nacheinander in *E. coli* CBTS (25) elektroporiert. Zur Bildung von Kointegraten wurden die Klone bei 30°C auf Agarplatten mit Chloramphenicol (12,5 µg/ml) und Kanamycin (50 µg/ml) gehalten, da der CBTS-Stamm nur bei 30°C das für die Rekombination essentielle recA-Protein exprimiert (25). Klone, in denen sich Kointegrate gebildet hatten, wurden bei 42°C auf Agarplatten mit Chloramphenicol (12,5 µg/ml) und Kanamycin (50 µg/ml) selektiert. Auflösung der Kointegrate wurde durch Inkubation der bakteriellen Klone bei 30°C auf Agarplatten ermöglicht, die nur Chloramphenicol enthielten (bei 30°C wird recA exprimiert, siehe oben). Zur Identifizierung von Klonen, in denen sich das Kointegrat aufgelöst hatte, wurden die bakteriellen Klone auf Agarplatten mit Chloramphenicol (12,5 µg/ml) und 5% Saccharose ausgestrichen und 1,5-2 Tage bei 30°C inkubiert. Bakterien, die das Kointegrat noch enthalten und somit das SacB-Protein exprimieren, können auf diesen Platten nicht wachsen (Negativ-Selektion); während dies für Klone mit aufgelöstem Kointegrat möglich ist. Die Auflösung des Kointegrates wurde durch Prüfung auf Kanamycin-Empfindlichkeit überprüft. Die Negativ-Selektion mit Hilfe des SacB-Gens auf saccharose-haltigen Agarplatten stellt im Vergleich zu dem in Beispiel 2 beschriebenen Verfahren einen wesentlichen Fortschritt dar, da Klone mit aufgelöstem Kointegrat sofort zu identifizieren sind.

Klone, die das mutante BAC-Plasmid enthalten, wurden schließlich aufgrund ihrer Tetrazyklin-Resistenz identifiziert (Inkubation auf Platten mit Chloramphenicol (12,5 µg/ml) plus Tetrazyklin (10 µg/ml)).

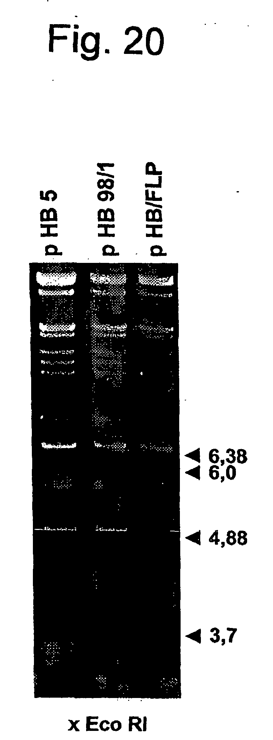

Das BAC-Plasmid aus einem der tetrazyklin-resistenten Klone wurde isoliert, mit EcoRI verdaut und das Spaltmuster mit dem EcoRI-Verdau des Ausgangsplasmids pHB5 verglichen (Fig. 20). Das EcoRI-Spaltmuster der beiden BAC-Plasmide ist bis auf die erwarteten Veränderungen identisch, d.h. außerhalb des mutierten Bereichs erfolgten keine Veränderungen. Wie erwartet, ist in BAC-Plasmid pHB98/1 eine 6.38 kb Bande verschwunden. Dafür entstehen zwei neue EcoRI-Banden von 4,88 und 3,70 kb (siehe Fig. 20), da die inserierte Tetrazyklin-Kassette eine zusätzliche EcoRI-Spaltstelle enthält (vgl. Fig. 17 unten).

Das Beispiel zeigt, daß das HCMV-BAC-Plasmid gezielt mutiert werden kann und daß das Verfahren geeignet ist, mutante HCMV-Genome herzustellen.

Das mutierte HCMV-BAC-Plasmid pHB98/1 wurde zusammen mit einem pp71-Expressionsplasmid in MRC5-Zellen transfiziert und führte zur Plaquebildung. Das pHB98/1-BAC-Plasmid ist also infektiös und führt nach Transfektion in permissive Zellen zur Bildung von mutanten Viren. Folglich ist das UL37-Gen für die Replikation von HCMV nicht essentiell.

Die Tetrazyklin-Kassette kann nach der Mutagenese durch Expression der FRTspezifischen FLP-Rekombinase in dem BAC-haltigen *E. coli*-Klon wieder deletiert werden, da die Tetrazyklin-Kassette nur benutzt wurde, um die mutanten Klone schneller und leichter identifizieren zu können. Die Verwendung der Tetrazyklin-Kassette ist nicht essentiell (vgl. Beispiel 2), unter dem genannten Gesichtpunkt aber hilfreich. Zur Deletion der Tetrazyklin-Kassette wurde das Plasmid pCP20 (32) in *E*. *coli*-Bakterien transformiert, die das pHB98/1 BAC-Plasmid enthielten. pCP20 verfügt über einen temperatursensitiven Replikationsmechanismus und exprimiert die FLP-Rekombinase (32). Die Exzision der Tetrazyklin-Kassette erfolgte während der Inkubation der *E. coli*-Bakterien bei 30°C. Anschließend werden die Bakterien auf Agarplatten mit Chloramphenicol (12,5 µg/ml) ausgestrichen und bei 43°C inkubiert, um das pCP20-Plasmid wieder zu verlieren (32). Screenen der Bakterien-Klone auf Tetrazyklin-Empfindlichkeit zeigte, daß in einem Großteil der Klone die Tetrazyklin-Kassette aus dem BAC-Plasmid herausgeschnitten worden war. Isolierung der BAC-Plasmide und Verdau mit EcoRI zeigte eine zusätzliche 6.0 kb EcoRI-Bande und den Verlust der 3.7 und 4.88 kb EcoRI-Banden und bestätigte somit das erfolgreiche Ausschneiden der Tetrazyklin-Kassette (siehe Figur 17 unten sowie Figur 20, Spur pHB/FLP).

### Beispiel 7: Direkte Transposon-Mutagenese des infektiösen MCMV-BAC-Plasmids

Transposons sind "springende" genetische Elemente, die vorwiegend in Bakterien gefunden werden. Sie enthalten in der Regel die für die Transposition notwendigen Enzyme sowie ein oder mehrere Resistenz-Gene. Modifizierte Transposons können für eine randomisierte Mutagenese von Bakterien-Genomen und Plasmiden verwendet werden. Sie inserieren an zufälliger Stelle in die DNA und unterbrechen dadurch den offenen Leserahmen des Gens an der Insertionsstelle. Dieses Mutagenese-Verfahren wurde hier erstmals für die direkte Mutagenese von BACs verwendet.

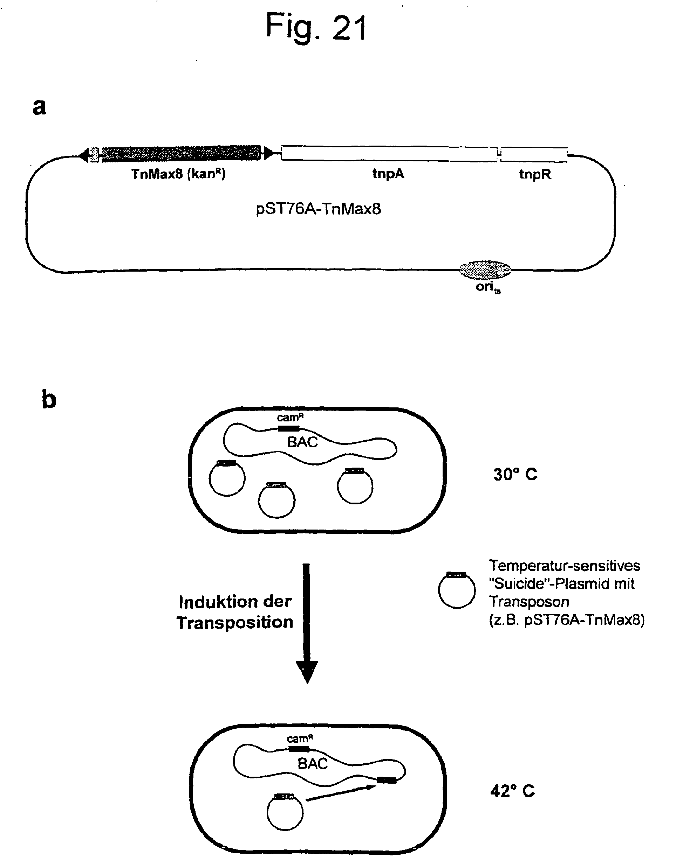

Basierend auf dem Transposon TnMax8, einem Derivat von Tn1721 (35) wurde das Plasmid pST76A-TnMax8 konstruiert. Dazu wurde pTnMax8 (enthält ein Kanamycin-Resistenzgen) und der Vektor pST76-A (34) mit dem Restriktionsenzym Pstl linearisiert und durch Ligation zu pST76A-TnMax8 fusioniert (Fig. 21a). Dieses Plasmid besitzt einen temperatursensitiven Replikationsmechanismus, der es ihm erlaubt, bei 30°C in *E. coli* zu replizieren, nicht jedoch bei 42°C. Für die direkte Transposon-Mutagenese des MCMV-Genoms wurden DH5a-Bakterien mit dem MCMV-BAC und pST76A-TnMax8 transformiert und bei 30°C kultiviert (Fig. 21b). Eine Transposition des Transposons findet nach Induktion der Transpositionsenzyme oder auch spontan statt. Werden diese Bakterien auf einer LB-Agarplatte ausgestrichen und bei 42°C in Anwesenheit von Chloramphenicol und Kanamycin inkubiert, so wachsen nur diejenigen Bakterien zu Kolonien heran, bei denen eine Transposition stattgefunden hat und somit die Kanamycin-Resistenz übertragen wurde (Fig. 21b). Das Plasmid pST76A-TnMax8 kann aufgrund des temperatursensitiven Replikationsmechanismus bei 42°C nicht replizieren und geht deshalb verloren. Das MCMV-BAC-Plasmid repliziert unabhängig von der Temperatur, und auf seine Anwesenheit wird mittels Chloramphenicol selektiert. Aufgrund der hohen Präferenz der Tn1721-Derivate für negativ verdrillte Plasmide führt eine Transposition überwiegend zur Insertion in das BAC, nur selten in das bakterielle Genom.

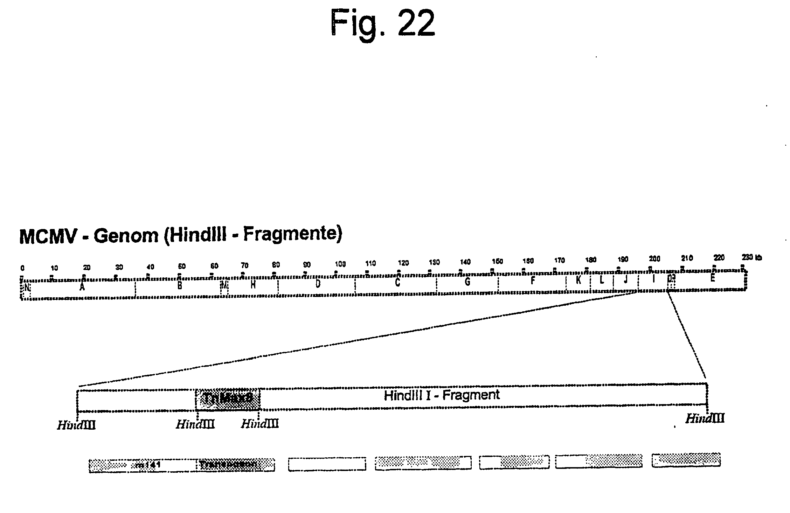

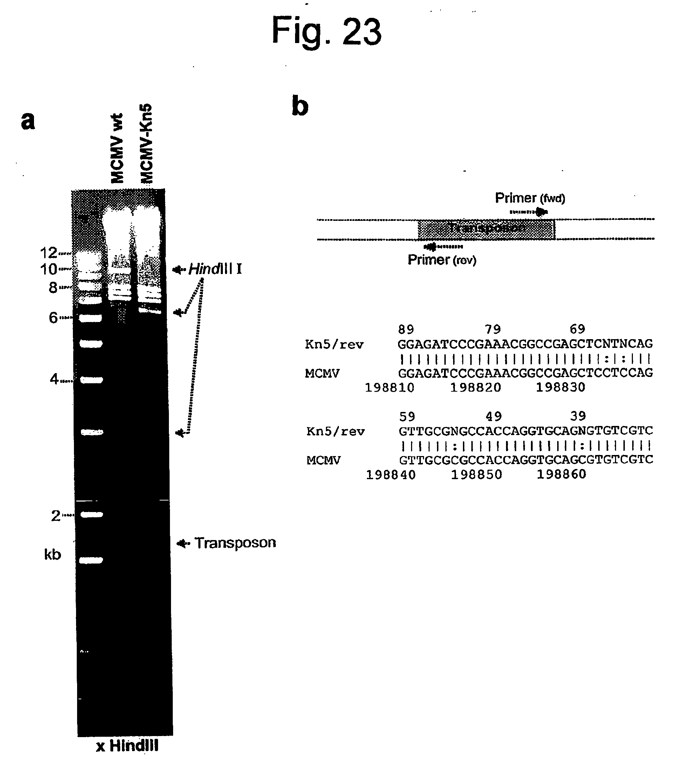

Die Charakterisierung einer MCMV-Transposon-Mutante soll hier exemplarisch anhand des Klons MCMV-Kn5 demonstriert werden: Um die Insertionsstelle des Transposons im BAC einzugrenzen, kann man Veränderungen des Restriktions-Musters nach Restriktionsverdau (hier mit Hindill) und Gelelektrophorese analysieren. Im vorliegenden Fall sieht man im Vergleich zum MCMV-Wildtyp (wt) ein Verschwinden der 9,5 kb Hindill I-Bande. Sie wird durch das Transposon in zwei Subfragmente von ca. 6,4 und 3,1 kb unterieilt (Fig. 22, Fig. 23a). Zusätzlich erkennt man im HindIII-Verdau eine Transposon-spezifische Bande von 1,8 kb (Fig. 23a). Durch direkte Ansequenzierung des Klons MCMV-Kn5 mit Primern, die an den Enden des Transposons binden, konnte die Insertionsstelle etwa bei Nukleotidposition 198900 lokalisiert werden (Figur 23b zeigt ein Alignment der erhaltenen Sequenz mit dem MCMV-Genom). Dies entspricht einer Insertion in das Gen m141 (Fig. 22).

Durch Transfektion von MCMV-Kn5-DNA in NIH-3T3-Fibroblasten wurde infektiöses Virus rekonstituiert. Bei diesem Virus ist das m141-Gen durch die Insertion des Transposons inaktiviert. Dies konnte durch Restriktionsverdau isolierter Virus-DNA gezeigt werden, wobei sich die gleiche Veränderung wie in Figur 23a zeigte. Damit wurde gleichzeitig nachgewiesen, daß das Gen m141 nicht essentiell für das Virus ist.

Das oben beschriebene Verfahren wurde auch schon erfolgreich zur Mutagenese des klonierten HCMV-Genoms angewandt (Daten nicht abgebildet). Zusätzlich zu dem Transposon-Vektor pST76A-TnMax8 wurden weitere Derivate mit Tetrazyklin- und Erythromycin-Resistenzgenen konstruiert. Damit lassen sich durch sequentielle Transposon-Mutagenese Mutanten mit zwei oder drei Gen-knockouts herstellen. Auch andere Transposons können in ähnlicher Weise für die direkte Mutagenese von klonierten Virusgenomen herangezogen werden.

Durch Veränderung des verwendeten Transposons lassen sich zusätzlich weitere Funktionen einfügen: So kann man durch Einfügen eines Indikator-Gens (z.B. IRES-EGFP) die temporale Expression der ausge'knock'ten Gene verfolgen. Durch Einsetzen von Sequenz-"Tags" in das Transposon lassen sich auch Virulenzgene in vivo identifizieren (36).

### Beispiel 8: Klonierung des Genoms des HCMV-Stamms Toledo als bakterielles künstliches Chromosom (BAC)

Der HCMV-Stamm Toledo verfügt im Vergleich zu dem Laborstamm AD169 über mindestens 19 zusätzliche Gene (4). Der Stamm Toledo hat hohe Ähnlichkeit zu HCMV-Patientenisolaten und stellt den Prototyp eines HCMV-Wildtyp-Stamms dar (4). Es war deshalb wichtig zu zeigen, daß sich Genomsequenzen aus einem HCMV-Patientenisolat zur Herstellung des erfindungsgemäßen rekombinanten Vektors ebenfalls eignen.

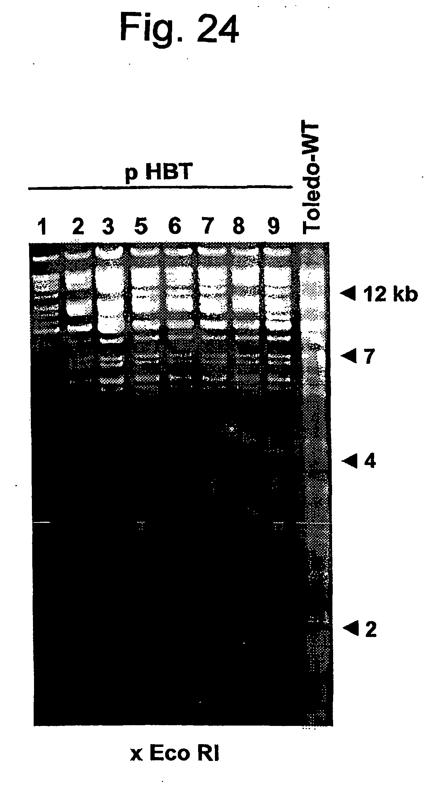

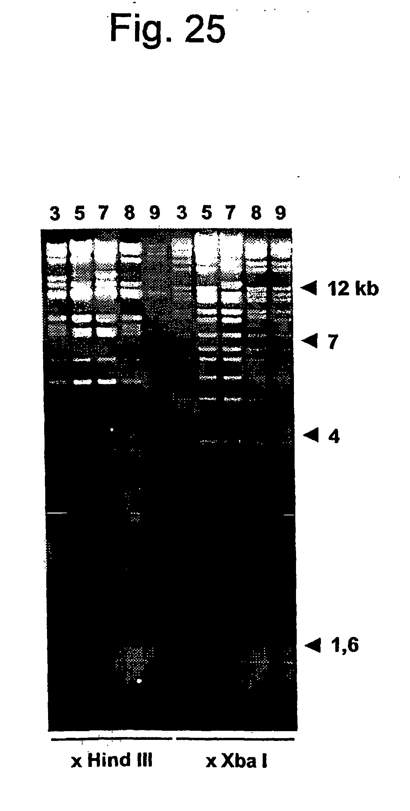

Für die Herstellung des Toledo-BAC-Plasmids wurde das in Beispiel 5 beschriebene Rekombinationsplasmid verwendet. Wie in Beispiel 5 beschrieben wurden ca. 30 µg des Xcml-linearisierten Rekombinationsplasmids in humane VorhautFibroblasten elektroporiert. Ca. 24 Stunden nach Elektroporation wurden die HFF-Zellen mit dem HCMV-Stamm Toledo mit einer moi von 1 infiziert. Das Anreicherungsverfahren mit Mykophenolsäure und Xanthin wurde wie in Beispiel 5 beschrieben durchgeführt. Schließlich wurde zirkuläre Virus-DNA aus den infizierten HFF-Zellen isoliert und in *E. coli* DH10B elektroporiert. Dann wurde Plasmid-DNA aus mehreren bakteriellen Klonen isoliert, mit EcoRI verdaut und das Spaltmuster mit dem EcoRI-Spaltmuster der Toledo-Virus-DNA verglichen (Figur 24). Das Spaltmuster der BAC-Klone ist weitgehend identisch mit dem der Toledo-Virus-DNA, d.h. die BAC-Plasmide enthalten bis auf die Integrationsstelle des BAC-Vektors im US2-US6-Bereich das vollständige Toledo-Genom. Das EcoRI-Muster der verschiedenen BAC-Plasmide zeigt geringe Unterschiede einzelner Fragmente im Bereich zwischen ca. 9,5 und 13 kb. Dieser Polymorphismus erklärt sich zum einen durch die unterschiedlichen isomeren Formen der HCMV-Toledo-Genome (vgl. Beispiel 5 und Figur 14), zum anderen kann auch die Zahl der a-*Repeats* variieren (3), wodurch sich die Länge einzelner EcoRI-Fragmente ändert. Figur 25 zeigt die HindIII- und Xbal-Spaltmuster einiger Toledo-BAC-Plasmide. Auch diese Muster sind bis auf die Größe einiger weniger Fragmente identisch.

Das Beispiel zeigt, daß sich das Verfahren zur Herstellung des erfindungsgemäßen rekombinanten Vektors für die Klonierung von Genomsequenzen verschiedener HCMV-Stämme, einschließlich HCMV-Patientenisolate) eignet.

### Literaturstellen:

1. Britt, W. J. & Alford, C. A. (1996) in: *Fields Virology,* Hrsg. Fields, B. N., Knipe, D. M. & Howley, P. M. (Lippincott-Raven, New York), S. 2493-2523.
2. Ho, M. (1991) in: *Cytomegalovirus: biology and infection,* Hrsg. Ho, M. (Plenum Publishing Corp., New York).
3. Chee, M.S., Bankier, A.T., Beck, S., Bohni, R., Brown, C. M., Cemy, R., Horsnell, T., Hutchison, C.A., Kouzarides, T., Martignetti, J. A., Preddie, E., Satchwell, S. C., Tomlinson, P., Weston, K. M. & Barrell, B. G. (1990) *Curr. Top*. *Microbiol. Immunol.* **154**, 125-169.
4. Cha, T.-A., Tom, B., Kembie, G. W, Duke, G. M., Mocarski, E.S. & Spate, R. R. (1996) *J*. *Virol.* **70**,78-83.
5. Rawlinson, W. D., Farrell, H. E. & Barrell, B. G. (1996) *J*. *Virol.* **70**, 8833-8849.
6. Roizman, B. & Sears, A. E. (1996) in: *Fields Virology*, Hrsg. Fields, B. N., Knipe, D M & Howley, P. M. (Lippincott-Raven, New York), S..2231-2295.
7. Spaete, R. & Mocarski, E.S. (1987) *Proc. Natl Acad. Sci. USA* **84**, 7213-7217.
8. Manning, W.C. & Mocarski, E.S. (1988) *Virology* **167**, 477-484.
9. Vieira, J., Farrell, H. E., Rawlinson, W. D. & Mocarski, E.S. (1994) *J. Virol*. **68**, 4837-4846.
10. Wolff, D., Jahn, G. & Plachter, B. (1993) *Gene* **130**, 167-173.
11. Greaves, R. F., Brown, J. M., Vieira, J. & Mocarski, E. S. (1995) *J*. *Gen. Virol.* **76**, 2151-2160,
12. van Zijl, M., Quint, W., Briaire, J., de Rover, T., Gielkens, A. & Berns, A. (1988) *J*. *Virol.* **62**, 2191-2195.
13. Kembie, G., Duke, G., Winter, R. & Spaete, R. (1996) *J*. *Virol.* **70**, 2044-2048.
14. Ebeling, A., Keil, G. M., Knust, E. & Koszinowski, U. H. (1983) *J*. *Virol*. **47**, 421-433.
15. Thale, R., Szepan, U., Hengel, H., Geginat, G., Lucin, P. & Koszinowski U. H. (1995) *J. Virol.* **69**, 6098-6105.
16. Messerle, M., Bühler, B, Keil, G. M. & Koszinowski. U. H. (1992) *J. Virol*. **66**,27-36.
17. Mercer, J. A., Marks, J. R. & Spector, D. H. (1983) *Virology* **129,** 94-106.
18. Hirt, B. (1967) *J*. *Mol. Biol.* **26,** 365-369.
19. Shizuya, H., Birren, B., Kim, U. J., Mancino, V., Slepak, T., Tachiiri, Y. & Simon, M. (1992) *Proc. Natl. Acad. Sci. USA* **89,** 8794-8797.
20. Maniatis, T., Fritsch, E. F. & Sambrook, J. (1989) *Motecular Cloning: A Laboratory Manual.* (Cold Spring Harbor Lab. Press, Plainview, NY).
21. Chang, A. C. & Cohen, S. N. (1978) *J*. *Bacteriol.* **134,**1141-56.
22. Sauer, B. (1993) *Methods Enzymol.* **225,** 890-900.
23. Keil, G. M., Ebeling-Keil, A. & Koszinowski, U. H. (1987) *J*. *Virol.* **56,** 526-533.
24. O'Connor, M., Peifer, M. & Bender, W. (1989) *Science* **244**,1307-1312.
25. Kempkes, B., Pich, D., Zeidler, R., Sugden, B. & Hammerschmidt, W. (1995) *J. Virol.* **69,** 231-238.
26. Mocarski, E. S. (1996) in: *Fields Virology,* Hrsg. Fields, B. N., Knipe, D. M. & Howley, P. M. (Lippincott-Raven, New York), S. 2447-2492.
27. Pfüller, R. & Hammerschmidt, W. (1996) *J. Virol.* **70,** 3423-3431.
28. Del Val, M., Volkmer, H., Rothbard, J. B., Jonjic, S., Messerle, M., Schickedanz, J., Reddehase, M. J. & Koszinowski, U. H. (1988) *J*. *Virol.* **62,** 3965-3972.
29. Virgin, H.W. 4th, Latreille, P., Wamsley, P., Hallsworth-K; Weck, K. E., Dal Canto, A. J., Speck, S. H. (1997) *J*. *Virol.* **71**, 5894-5904.
30. Simas, J. P., Bowden, R. J., Paige, V., Efstathiou, S. (1998) *J. Gen. Virol.* **79**, 149-153.
31. Jones, T. R., Hanson, K., Sun, L., Slater, J. S., Stenberg, R. M., Campbell, A. E. (1995) *J. Virol.* **69**, 4830-4841.
32. Cherepanov, P. P., Wackernagel, W. (1995) *Gene* **158**, 9-14.
33. Dargan, D. J., Jamieson, F. E., MacLean, J., Dolan, A., Äddison, C., McGeoch, D. J. (1997) *J*. *Virol.* **71**, 9833-9836.
34. Posfai, G, Koob, M. D., Kirkpatrick, H. A., Blattner, F. R. (1997) *J*. *Bacteriol.* **179**, 4426-4428.
35. Kahrs, A. F., Odenbreit, S., Schmitt, W., Heuermann, D., Meyer T. F., Haas, R. (1995) *Gene* **167**:53-57.
36. Hensel, M., Shea, J. E., Gleeson, C., Jones, M. D., Dalton, E., Holden, D. W. (1995) *Science* **269**:400-403.
37. Baldick, C. J. Jr., Marchini, A., Patterson, C. E., Shenk, T. (1997) *J. Virol.* **71**:4400-4408.

## Patentansprüche

1. Rekombinanter Vektor, der virale Genomsequenzen mit einer Größe von mehr als 100 kb sowie Sequenzen eines Klonierungsvehikels enthält, die die Fähigkeit zur DNA-Replikation in einer Wirtszelle besitzen, wobei das Klonierungsvehikel ein bakterielles künstliches Chromosom (BAC) ist, **dadurch gekennzeichnet, dass** die viralen Genomsequenzen infektiös sind und der Vektor nach dem Einbringen in eine geeignete permissive Zelle wie ein Virusgenom repliziert und verpackt werden kann.

2. Rekombinanter Vektor nach Anspruch 1, **dadurch gekennzeichnet, daß** die infektiösen viralen Genomsequenzen eine Größe von mehr als 200 kb haben.

3. Rekombinanter Vektor nach Anspruch 1 oder 2, **dadurch gekennzeichnet, daß** die viralen Genomsequenzen von einem DNA-Virus stammen.

4. Rekombinanter Vektor nach Anspruch 3, **dadurch gekennzeichnet, daß** die viralen Genomsequenzen von einem Herpes-Virus stammen.

5. Rekombinanter Vektor nach Anspruch 4, **dadurch gekennzeichnet, daß** die virale Genomsequenzen von einem Betaherpesvirus stammen.

6. Rekombinanter Vektor nach Anspruch 5, **dadurch gekennzeichnet, daß** die viralen Genomsequenzen vom menschlichen Cytomegalievirus stammen.

7. Rekombinanter Vektor nach Anspruch 5, **dadurch gekennzeichnet, daß** die viralen Genomsequenzen vom Maus-Cytomegalievirus stammen.

8. Rekombinanter Vektor nach Anspruch 4, **dadurch gekennzeichnet, daß** die virale Genomsequenzen von einem Gammaherpesvirus stammen.

9. Rekombinanter Vektor nach Anspruch 4, **dadurch gekennzeichnet, daß** die virale Genomsequenzen vom murinen Gammaherpesvirus 68 (MHV 68) stammen.

10. Rekombinanter Vektor nach einem oder mehreren der vorstehenden Ansprüche, **dadurch gekennzeichnet, daß** die Sequenzen des Klonierungsvehikels von identischen Sequenzabschnitten flankiert sind.

11. Rekombinanter Vektor nach einem oder mehreren der vorstehenden Ansprüche, **dadurch gekennzeichnet, daß** die Sequenzen des Klonierungsvehikels von Erkennungssequenzen für sequenzspezifische Rekombinasen und/oder von Restriktionsschnittstellen flankiert sind, die im restlichen Vektor nicht vorkommen.

12. Rekombinanter Vektor nach Anspruch 11, **dadurch gekennzeichnet, daß** die Erkennungssequenzen für sequenzspezifische Rekombinasen loxP-Stellen sind.

13. Rekombinanter Vektor nach einem oder mehreren der vorstehenden Ansprüche, **dadurch gekennzeichnet, daß** zusätzlich Selektions- und/oder Markergene enthalten sind.

14. Zelle, enthaltend einen rekombinanten Vektor nach einem der Ansprüche 1 bis 13.

15. Verfahren zur Herstellung eines rekombinanten Vektors gemäß einem oder mehreren der Ansprüche 1 bis 13, das folgende Schritte umfaßt:
a) Einbringen einer Sequenz (1), enthaltend Sequenzen eines Klonierungsvehikels, die die Fähigkeit zur DNA-Replikation in einer Wirtszelle besitzen, wobei das Klonierungsvehikel ein bakterielles künstliches Chromosom (BAC) ist, in eine Zelle, die infektiöse virale Genomsequenzen enthält, und
b) Rekombinieren der Sequenz (1) aus Schritt a) mit den viralen Genomsequenzen zum Erhalt eines rekombinanten Vektors.

16. Verfahren nach Anspruch 15, **dadurch gekennzeichnet, daß** der Schritt b) über homologe Rekombination erfolgt.

17. Verfahren nach Anspruch 15 oder 16, **dadurch gekennzeichnet, daß** als Zelle in Schritt a) eine eukaryontische Zelle verwendet wird.

18. Verfahren nach Anspruch 17, **dadurch gekennzeichnet, daß** die eukaryontischen Zellen Säugerzellen sind.

19. Verfahren nach Anspruch 18, **dadurch gekennzeichnet, daß** die eukaryontischen Zellen primäre Fibroblasten, humane Vorhautfibroblasten (HFF) oder embryonale Maus-Fibroblasten sind.

20. Verfahren nach Anspruch 19, **dadurch gekennzeichnet, daß** die eukaryontischen Zellen NIH3T3-Fibroblasten sind.

21. Verfahren nach einem der Ansprüche 15 bis 20, **dadurch gekennzeichnet, daß** die Sequenz (1) durch ein Calciumphosphat-Präzipitations-, ein Lipofektions- oder ein Elektroporationsverfahren in die Zellen eingebracht wird.

22. Verfahren nach einem der Ansprüche 15 bis 20, **dadurch gekennzeichnet, daß** die Sequenz (1) durch einen weiteren viralen Vektor in die Zellen eingebracht wird.

23. Verfahren nach Anspruch 15, **dadurch gekennzeichnet, daß** als Zelle in Schritt a) ein bakterieller Organismus verwendet wird.

24. Verfahren nach Anspruch 23, **dadurch gekennzeichnet, daß** als Zelle in Schritt a) *E. coli* verwendet wird.

25. Verwendung eines rekombinanten Vektors nach einem oder mehreren der Ansprüche 1 bis 13 zur Mutagenese der darin enthaltenen infektiösen viralen Genomsequenzen.

26. Verfahren zur Mutagenese von in einem rekombinanten Vektor gemäß einem oder mehreren der Ansprüche 1 bis 13 enthaltenen viralen Genomsequenzen, das folgende Schritte umfaßt:
A) Einbringen des rekombinanten Vektors in eine bakterielle Wirtszelle; und
B) Mutagenisieren der viralen Genomsequenzen.

27. Verfahren nach Anspruch 26, **dadurch gekennzeichnet, daß** die Mutagenisierung der viralen Genomsequenzen durch homologe Rekombination des rekombinanten Vektors mit in der bakteriellen Wirtszelle enthaltenen DNA-Molekülen erfolgt.

28. Verfahren nach Anspruch 27, **dadurch gekennzeichnet, daß** die homologe Rekombination des rekombinanten Vektors mit einem mutanten Allel erfolgt.

29. Verfahren nach Anspruch 26, **dadurch gekennzeichnet, daß** die Mutagenisierung der viralen Genomsequenzen mit Hilfe eines Transposons erfolgt.

30. Verfahren nach einem oder mehreren der Ansprüche 26 bis 29, **dadurch gekennzeichnet, daß** der rekombinante Vektor gemäß einem Verfahren nach einem oder mehreren der Ansprüche 15 bis 24 erhältlich ist.

31. Rekombinanter Vektor nach einem der Ansprüche 1 bis 13, bei dem die infektiösen viralen Genomsequenzen durch Mutagenese gemäß einem der Ansprüche 26 bis 30 verändert sind.

32. Rekombinanter Vektor nach Anspruch 31, **dadurch gekennzeichnet, daß** die veränderten viralen Genomsequenzen eine Größe von mehr als 200 kb haben.

33. Rekombinanter Vektor nach einem der Ansprüche 1 bis 13, 31 oder 32 als Medikament.

34. Verwendung des rekombinanten Vektors nach einem der Ansprüche 1 bis 13, 31 oder 32 zur Herstellung einer pharmazeutischen Zusammensetzung zur Durchführung von somatischer Gentherapie.

35. Verwendung des rekombinanten Vektors nach einem der Ansprüche 1 bis 13, 31 oder 32 zur Herstellung einer pharmazeutischen Zusammensetzung als Impfstoff.

## Claims

1. A recombinant vector containing viral genome sequences having a size larger than 100 kb, as well as sequences of a cloning vehicle which are capable of DNA replication in a host cell, with the cloning vehicle being a bacterial artificial chromosome (BAC), **characterized in that** the viral genome sequences are infectious and after introduction into a suitable permissive cell the vector can be replicated and packed like a virus genome.

2. The recombinant vector according to claim 1, **characterized in that** the infectious viral genome sequences have a size larger than 200 kb.

3. The recombinant vector according to claim 1 or 2, **characterized in that** the viral genome sequences derive from a DNA virus.

4. The recombinant vector according to claim 3, **characterized in that** the viral genome sequences derive from a herpesvirus.

5. The recombinant vector according to claim 4, **characterized in that** the viral genome sequences derive from a betaherpesvirus.

6. The recombinant vector according to claim 5, **characterized in that** the viral genome sequences derive from human cytomegalovirus.

7. The recombinant vector according to claim 5, **characterized in that** the viral genome sequences derive from mouse cytomegalovirus.

8. The recombinant vector according to claim 4, **characterized in that** the viral genome sequences derive from a gammaherpesvirus.

9. The recombinant vector according to claim 4, **characterized in that** the viral genome sequences derive from murine gammaherpesvirus 68 (MHV 68).

10. The recombinant vector according to one or several of the preceding claims, **characterized in that** the sequences of the cloning vehicle are flanked by identical sequence sections.

11. The recombinant vector according to one or several of the preceding claims, **characterized in that** the sequences of the cloning vehicle are flanked by recognition sequences for sequence-specific recombinases and/or by restriction sites which do not appear in the rest of the vector.

12. The recombinant vector according to claim 11, **characterized in that** the recognition sequences for sequence-specific recombinases are loxP sites.

13. The recombinant vector according to one or several of the preceding claims, **characterized in that** selection and/or marker genes are additionally contained.

14. A cell containing a recombinant vector according to any one of claims 1 to 13.

15. A method for producing a recombinant vector according to one or several of claims 1 to 13, comprising the following steps:
a) introducing a sequence (1), containing sequences of a cloning vehicle which are capable of DNA replication in a host cell, with the cloning vehicle being a bacterial artificial chromosome (BAC), into a cell containing infectious viral genome sequences, and
b) recombining sequence (1) of step a) with the viral genome sequences to obtain a recombinant vector.

16. The method according to claim 15, **characterized in that** step b) is carried out via homologous recombination.

17. The method according to claim 15 or 16, **characterized in that** a eukaryotic cell is used as the cell in step a).

18. The method according to claim 17, **characterized in that** the eukaryotic cells are mammalian cells.

19. The method according to claim 18, **characterized in that** the eukaryotic cells are primary fibroblasts, human foreskin fibroblasts (HFF) or mouse embryonic fibroblasts.

20. The method according to claim 19, **characterized in that** the eukaryotic cells are NIH3T3 fibroblasts.

21. The method according to any one of claims 15 to 20, **characterized in that** sequence (1) is introduced into the cells by a calcium phosphate precipitation method, a lipofection method or an electroporation method.

22. The method according to any one of claims 15 to 20, **characterized in that** sequence (1) is introduced into the cells by a further viral vector.

23. The method according to claim 15, **characterized in that** a bacterial organism is used as the cell in step a).

24. The method according to claim 23, **characterized in that** *E*. *coli* is used as the cell in step a).

25. Use of a recombinant vector according to one or several of claims 1 to 13 for the mutagenesis of the infectious viral genome sequences contained therein.

26. A method for the mutagenesis of viral genome sequences contained in a recombinant vector according to one or several of claims 1 to 13, the method comprising the following steps:
A) introducing the recombinant vector into a bacterial host cell; and
B) mutagenizing the viral genome sequences.

27. The method according to claim 26, **characterized in that** the mutagenesis of viral genome sequences is carried out by homologous recombination of the recombinant vector with DNA molecules contained in the bacterial host cell.

28. The method according to claim 27, **characterized in that** the homologous recombination of the recombinant vector is carried out with a mutant allele.

29. The method according to claim 26, **characterized in that** the mutagenesis of the viral genome sequences is carried out with the help of a transposon.

30. The method according to one or several of claims 26 to 29, **characterized in that** the recombinant vector is obtainable by a method according to one or several of claims 15 to 24.

31. The recombinant vector according to any one of claims 1 to 13, in which the infectious viral genome sequences are modified by mutagenesis according to any one of claims 26 to 30.

32. The recombinant vector according to claim 31, **characterized in that** the modified viral genome sequences have a size larger than 200 kb.

33. The recombinant vector according to any one of claims 1 to 13, 31 or 32 as a drug.

34. Use of the recombinant vector according to any one of claims 1 to 13, 31 or 32 for preparing a pharmaceutical composition for performing somatic gene therapy.

35. Use of the recombinant vector according to any one of claims 1 to 13, 31 or 32 for preparing a pharmaceutical composition as a vaccine.

## Revendications

1. Vecteur recombinant, qui contient des séquences de génome viral ayant une taille supérieure à 100 kb, ainsi que des séquences d'un véhicule de clonage, qui ont la capacité de procéder à une réplication de l'ADN dans une cellule hôte, le véhicule de clonage étant un chromosome bactérien artificiel (BAC), **caractérisé en ce que** les séquences de génome viral sont infectieuses et **en ce que** le vecteur, après introduction dans une cellule permissive appropriée, peut être répliqué et empaqueté comme un génome.

2. Vecteur recombinant selon la revendication 1, **caractérisé en ce que** les séquences infectieuses de génome viral ont une taille supérieure à 200 kb.

3. Vecteur recombinant selon la revendication 1 ou 2, **caractérisé en ce que** les séquences de génome viral proviennent d'un virus à ADN.

4. Vecteur recombinant selon la revendication 3, **caractérisé en ce que** les séquences de génome viral proviennent d'un virus de l'herpès.

5. Vecteur recombinant selon la revendication 4, **caractérisé en ce que** les séquences de génome viral proviennent d'un virus du bêtaherpès.

6. Vecteur recombinant selon la revendication 5, **caractérisé en ce que** les séquences de génome viral proviennent d'un cytomégalovirus humain.

7. Vecteur recombinant selon la revendication 5, **caractérisé en ce que** les séquences de génome viral proviennent d'un cytomégalovirus de souris.

8. Vecteur recombinant selon la revendication 4, **caractérisé en ce que** les séquences de génome viral proviennent d'un virus de gammaherpès.

9. Vecteur recombinant selon la revendication 4, **caractérisé en ce que** les séquences de génome viral proviennent du virus de gammaherpès murin 68(MHV 68).

10. Vecteur recombinant selon l'une ou plusieurs des revendications précédentes, **caractérisé en ce que** les séquences du véhicule de clonage sont flanquées de segments de séquence identiques.

11. Vecteur recombinant selon l'une ou plusieurs des revendications précédentes, **caractérisé en ce que** les séquences du véhicule de clonage sont flanquées de séquences de reconnaissance pour des recombinases spécifiques de séquence et/ou des interfaces de restriction, qui n'apparaissent pas dans le reste du vecteur.

12. Vecteur recombinant selon la revendication 11, **caractérisé en ce que** les séquences de reconnaissance pour des recombinases spécifiques de séquence sont des sites loxP.

13. Vecteur recombinant selon l'une ou plusieurs des revendications précédentes, **caractérisé en ce qu'**il contient en outre des gènes de sélection et/ou marqueurs.

14. Cellule contenant un vecteur recombinant selon l'une des revendications 1 à 13.

15. Procédé de préparation d'un vecteur recombinant selon l'une ou plusieurs des revendications 1 à 13, qui comprend les étapes suivantes :
a) introduction d'une séquence (1), contenant des séquences d'un véhicule de clonage qui présente la capacité de procéder à une réplication de l'ADN dans une cellule hôte, le véhicule de clonage étant un chromosome bactérien artificiel (BAC), dans une cellule qui contient des séquences infectieuses de génome viral, et
b) recombinaison de la séquence (1) de l'étape a) avec les séquences de génome viral, pour obtenir un vecteur recombinant.

16. Procédé selon la revendication 15, **caractérisé en ce que** l'étape b) est réalisée par une recombinaison homologue.

17. Procédé selon la revendication 15 ou 16, **caractérisé en ce qu'**on utilise en tant que cellule dans l'étape a) une cellule d'eucaryote.

18. Procédé selon la revendication 17, **caractérisé en ce que** les cellules d'eucaryote sont des cellules de mammifère.

19. Procédé selon la revendication 18, **caractérisé en ce que** les cellules d'eucaryote sont des fibroblastes primaires, des fibroblastes de prépuce humain (HFF) ou des fibroblastes d'embryon de souris.

20. Procédé selon la revendication 19, **caractérisé en ce que** les cellules d'eucaryote sont des fibroblastes NIH3T3.

21. Procédé selon l'une des revendications 15 à 20, **caractérisé en ce que** la séquence (1) est introduite dans les cellules par un procédé de précipitation au phosphate de calcium, par un procédé de lipofection ou par un procédé d'électroporation.

22. Procédé selon l'une des revendications 15 à 20, **caractérisé en ce que** la séquence (1) est introduite dans les cellules par un autre vecteur viral.

23. Procédé selon la revendication 15, **caractérisé en ce qu'**on utilise en tant que cellule dans l'étape a) un organisme bactérien.

24. Procédé selon la revendication 23, **caractérisé en ce qu'**on utilise en tant que cellule dans l'étape a) *E*. *coli*.

25. Utilisation d'un vecteur recombinant selon l'une ou plusieurs des revendications 1 à 13 pour la mutagenèse des séquences de génome viral infectieuses qui y sont contenues.

26. Procédé de mutagenèse de séquences de génome viral contenues dans un vecteur recombinant selon l'une ou plusieurs des revendications 1 à 13, qui comprend les étapes suivantes :
A) introduction du vecteur recombinant dans une cellule hôte bactérienne ; et
B) mutagénisation des séquences de génome viral.

27. Procédé selon la revendication 26, **caractérisé en ce que** la mutagénisation des séquences de génome viral est réalisée par recombinaison homologue du vecteur recombinant avec les molécules d'ADN contenues dans la cellule hôte bactérienne.

28. Procédé selon la revendication 27, **caractérisé en ce que** la recombinaison homologue du vecteur recombinant s'effectue avec un allèle mutant.

29. Procédé selon la revendication 26, **caractérisé en ce que** la mutagénisation des séquences de génome viral est réalisée à l'aide d'un transposon.

30. Procédé selon l'une ou plusieurs des revendications 26 à 29, **caractérisé en ce que** le vecteur recombinant peut être obtenu par un procédé selon l'une ou plusieurs des revendications 15 à 24.

31. Vecteur recombinant selon l'une des revendications 1 à 13, dans lequel les séquences infectieuses de génome viral sont modifiées par une mutagénèse selon l'une des revendications 26 à 30.

32. Vecteur recombinant selon la revendication 31, **caractérisé en ce que** les séquences de génome viral ayant été modifiées ont une taille supérieure à 200 kb.

33. Vecteur recombinant selon l'une des revendications 1 à 13, 31 et 32, en tant que médicament.

34. Utilisation du vecteur recombinant selon l'une des revendications 1 à 13, 31 et 32, pour préparer une composition pharmaceutique destinée à la mise en oeuvre d'une thérapie génique somatique.

35. Utilisation du vecteur recombinant selon l'une des revendications 1 à 13, 31 et 32, pour préparer une composition pharmaceutique servant de vaccin.
